# EUROPEAN PATENT SPECIFICATION

(11) **EP 0 528 970 B1**
(45) Date of publication and mention of the grant of the patent: **07.07.1999**
(21) Application number: 91910496.8
(22) Date of filing: 25.04.1991
(51) Int. Cl.: C12N 15/82, C12N 15/11, C12N 15/31, C12N 15/52, C12N 5/04

(54) **METHODS AND COMPOSITIONS FOR MODULATING ETHYLENE LEVELS IN PLANT TISSUES**
METHODEN UND REAGENTIEN UM DAS ETHYLENNIVEAU IN PFLANZEN ZU MODULIEREN
PROCEDES ET COMPOSITIONS DE MODULATION DE NIVEAUX D'ETHYLENE DANS DES TISSUS VEGETAUX

(30) Priority: 26.04.1990 US 514029
(43) Date of publication of application: 03.03.1993
(73) Proprietor: Calgene LLC, Davis, California 95616 (US); The Regents of the University of California, Oakland, CValifornia 94607-5200 (US)
(72) Inventor: YANG, Shang, Fa, Davis, CA 95616 (US); HIATT, William, R., Davis, CA 95616 (US)
(74) Representative: Goldin, Douglas Michael
(86) International application number: US9102958
(87) International publication number: WO9116417

(56) References cited:
- WO-A-91/09112
- WO-A-92/12249
- JOURNAL OF BIOLOGICAL CHEMISTRY, vol. 262, no. 33, 25 November 1987, Baltimore, MD (US); C.W. TABOR et al., pp. 16037-16404
- JOURNAL OF EXPERIMENTAL BOT., 1990 Annual Meeting of the Society for Experimental Biology, vol. 41, no. SUP., 1990; A.J. HAMILTON et al., p. P5-5
- THE PLANT CELL, vol. 3, no. 3, March 1991, Rockville, MD (US); M.F. DILWORTH et al., pp. 213-218
- AGRICULTURAL & BIOLOGICAL CHEMISTRY, vol. 42, no. 10, 1978; HONMA et al., pp. 1825-1831
- COLD SPRING HARBOUR SYMPOSIA ON QUANTITATIVE BIOLOGY, vol. 51, 1986; MULLIS et al., pp. 263-273
- METHODS IN ENZYMOLOGY, vol. 152, November 1987; WALLACE et al., pp. 432-442
- METHODS IN ENZYMOLOGY, vol. 152, November 1987; HELFMAN et al., pp. 451-457
- SCIENCE, vol. 227, 08 March 1985; HORSCH et al., pp. 1229-1231
- THOMSON et al., "Plant Senescence: Its Biochemistry & Physiology", 1987, American Society of Plant Physiologists (publ.); pp. 156-166
- PROCEEDINGS OF THE NATL. ACADEMY OF SCIENCES USA, vol. 86, September 1989, Washington, DC (US); SATO et al., pp. 6621-6625
- AGRICULTURAL & BIOLOGICAL CHEMISTRY, vol. 50, no. 12, 1986; HONMA, pp. 3189-3190
- BIOCHEMISTRY, vol. 20, 1981; WALSH et al., pp. 7509-7518
- PROCEEDINGS OF THE NATL. ACADEMY OF SCIENCES USA, vol. 76, no. 1, January 1979, Washington, DC (US); ADAMS et al., pp. 170-174

## Description

### Technical Field

This invention relates to modulating ethylene levels to alter growth related characteristics of plant tissues.

### Background

Ethylene (CH₂=CH₂) is a gaseous hormone implicated in the natural regulation of tissue growth and development in higher plants. It is regularly produced by plant cells and diffuses out of the plant tissue in a manner similar to carbon dioxide. Plant cells produce low levels of ethylene at all times, and these levels may effect normal growth and development. Depending upon the growth stage of the plant, various plant parts may demonstrate specialized effects as a result of the exposure to ethylene.

Increased levels of ethylene production are observed during particular stages of plant development. These sharp increases in ethylene are seen contemporaneous with specific plant processes, and in particular, seed germination, pollination, wounding and a variety of other stresses, fruit ripening, leaf and petal senescence, and abscission. In general, all of these processes result in an increase in the levels of hydrolytic enzymes. During processes such as fruit ripening, flower senescence and abscission, ethylene production is described as "autocatalytic." In other words, exposure to ethylene dramatically augments the amount of ethylene produced by the subject tissue and the sensitivity of that tissue to the ethylene-mediated effect. Ethylene is "applied" from the build-up of released ethylene which results from the increase in ethylene production, as well as the external application of ethylene from other contemporaneous plant tissue, as ethylene gas, or as chemical compositions which release ethylene. Tissues unrelated to the given "ethylene-sensitive" tissue remain relatively unaffected by the exposure to increased ethylene. Thus, it is postulated that other hormonal influences are involved regarding ethylene susceptibility in the first instance.

To some extent, modern agricultural and horticultural practices exploit the properties of ethylene. Exposure to tissue emitting increased ethylene levels is avoided to prevent the sympathetic triggering of undesirable changes in the remainder of ethylene-susceptible tissue. Thus, cut flowers are treated with silver ions in the form of silver thiosulfate to delay senescense effects observed by the rise in ethylene production. In a like fashion, unripened climacteric fruits are maintained in well-ventilated storage until use. In the case of tomato, when ripening is desired, the fruit is treated with exogenous ethylene or ethylene gas or ethylene-releasing compounds such as ethephon (2-chloroethanephosphonic acid).

With the advent of genetic engineering, various strategies have been proposed to provide the regulation of ethylene production in plants, especially to prevent increased ethylene production and accumulation. In particular, antisense of various genes involved in the biosynthesis of ethylene may provide mechanisms to regulate ethylene production. However, antisense technology requires the use of DNA sequence with a high degree of complementarity to the gene of interest. Slight differences in the respective ethylene synthesis pathway of different crops may frustrate the ability to effectively provide desired phenotypic changes with a minimum of effort. Because such methods are limited to the specific gene(s) of interest and, often, the specific plant itself, a method is needed which will be universally applicable to a wide variety of crops. In addition, the control of a rate limiting precursor of ethylene biosynthesis is especially desired.

### Relevant Literature

The role of ethylene and ethylene synthesis in fruit ripening is described in *Plant Senescence: Its Biochemistry and Physiology,* eds. Thompson *et al.* (1987) pp. 156-166, and the role of ethylene in other aspects of plant development is described in *Ethylene and Plant Development,* eds. Roberts and Tucker (1985). In 1979, Adams and Yang, *PNAS USA* (1979) *76*:170-174, reported that ethylene is biosynthesized from 1-Aminocyclopropane-1-carboxylic acid (ACC) and that the level of ACC in plant tissues controls the rate of ethylene production. ACC is synthesised by ACC synthase (SAMase), and the zucchini ACC synthase has been cloned and expressed (Sato & Theologis (1989), PNAS USA, 86, 6621-6625). ACC deaminase, which catalyzes the cleavage of ACC to ammonia and alpha-ketobutyrate, has been purified from a *Pseudomonas* sp. and a yeast *Hansenula saturnus,* which use ACC as a sole nitrogen source. Honma and Shimomura *Agric. Biol. Chem.* (1978) *42*:1825-1831.

### SUMMARY OF THE INVENTION

Novel compositions and methods are provided for modifying the ethylene levels of plant tissues of interest, including petal, leaf and various fruit tissues. The methods involve transforming a plant cell of interest with an expression cassette functional in a plant cell comprising a transcriptional and translational initiation regulatory region, joined in reading frame 5' to a DNA sequence encoding an ACC deaminase enzyme capable of modulating the production of ethylene, and translational and transcriptional termination regions. Expression of the enzyme provides for a decrease in ethylene production as a result of altered concentrations of substrate for enzyme involved in ethylene biosynthesis. Of particular interest is the selective control of ethylene production in plant tissues such as flowers, fruit and leaves, especially to inhibit dramatic increases in ethylene production related to fruit and tissue senescence, abscission and/or ripening. The invention therefore provides:

A DNA construct comprising:
in the 5'-3' direction of transcription, the following components, a transcriptional and translational initiation region functional in a plant cell, a DNA sequence encoding a 1-aminocyclopropane-1-carboxylic acid (ACC) deaminase, which deaminase is capable of metabolising 1-aminocyclopropane-1-carboxylic acid (ACC) and thus reducing ethylene biosynthesis and a transcriptional termination region, wherein said components are operably linked and wherein at least one of said initiation and termination regions is other than the region naturally associated with said DNA sequence;
a method for reducing ethylene levels in plant tissue comprising: transforming a plant cell with a DNA sequence encoding 1-aminocyclopropane carboxylic acid (ACC) deaminase, thereby to obtain plant tissue comprising one or more plant cells which comprise a DNA sequence encoding a 1-aminocyclopropane-1-carboxylic acid (ACC) deaminase, which deaminase is capable of metabolizing l-aminocyclopropane-l-carboxylic acid (ACC) and thus reducing ethylene biosynthesis; and growing said tissue under conditions whereby said gene is expressed and the ethylene level of said plant tissue is reduced compared to the level normally present in said plant tissue;
a plant cell comprising a DNA construct according to the invention;
a plant cell comprising a bacterial 1-aminocyclopropane-1 carboxylic acid (ACC) deaminase;

A method of producing a 1-aminocyclopropane carboxylic acid (ACC) deaminase, which deaminase is capable of metabolizing 1-aminocyclopropane-1-carboxylic acid (ACC), and thus reducing the level of ethylene present in a host cell comprising:
transforming a host cell with a DNA construct according to any one of claims 1 to 9 and growing said cell under conditions which will permit the production of said deaminase; and
a method for altering ethylene levels in a plant comprising:
transforming a plant cell with a DNA sequence encoding 1-aminocyclopropane carboxylic acid (ACC) deaminase; obtaining a plant comprising one or more cells comprising said DNA sequence; and expressing said DNA sequence in said plant, whereby expression of said DNA sequence reduces the ACC available for ethylene conversion in said plant.

### BRIEF DESCRIPTION OF THE DRAWINGS

Figure 1 shows the amino acid (SEQ ID NOS: 1-4) and corresponding DNA sequence (SEQ ID NOS: 5-8) of oligonucleotides ACDD 1-4 used as primers for PCR reactions.

Figure 2 shows the complete DNA sequence (SEQ ID NO: 9) of the ACC deaminase coding region as well as 623 bp of 5' untranslated region and 143 bp of 3' untranslated region. The 1-letter amino acid designation is shown under each codon of the coding region.

Figure 3 shows a partial restriction map of pCGN1479 which contains an ACC deaminase genomic clone.

### DESCRIPTION OF THE SPECIFIC EMBODIMENTS

In accordance with the subject invention, novel DNA sequences, DNA constructs, methods and compositions are provided which permit modification of the ethylene concentration of plant products by means of increased metabolism of a substrate for ethylene biosynthesis in a tissue of interest. Plant cells are transformed with an expression cassette comprising a DNA sequence encoding an ACC deaminase enzyme capable of decreasing the amount of an ethylene biosynthesis precursor in the tissue of interest. Desirably, integration constructs may be prepared which provide for integration of the expression cassette into the genome of a plant host. Depending upon the desired application, it may be useful to preferentially express the enzyme in the tissue of interest. Tissue specificity may be accomplished by the use of transcriptional regulatory regions having the desired expression profile.

Thus, by this invention, increased production of ethylene may be controlled so as to provide a means to delay deleterious physiological changes brought on by the increased production and exposure to ethylene. Desirably, such affects will be maintained during the respective period of ethylene accumulation of interest and not affect hormonal activities associated with low levels of ethylene. In other words, minimal impact to other plant processes is desired. For example, by reducing ethylene concentrations in the petals and/or leaves of cut flowers, the plants will not wilt as quickly. Plants of interest include petunia, rose, carnation, gerbera, chrysanthemum and all other horticultural crops of interest. In a like manner, vegetable quality may be enhanced, especially, when applied to green and/or leafy vegetables such as broccoli, kale, parsley, and spinach and to other fruit and vegetables such as carrots, cucumber, citrus fruit and grain crops to mitigate effects of yellowing or wilting. Control of ethylene exposure has also been demonstrated to prevent deterioration of fruit and vegetables such as tomato, cucumber, kiwi fruit, banana, melons, apples, avocado, and pear. Other applications related to increased ethylene production, such as early abortion of flower, leaf, and fruit tissue in general, including fruit which is not considered climacteric such as citrus, and especially as to developmentally related fruit structures such as seed pod shatter observed in rapeseed varieties, and boll-drop in cotton, should also respond to ethylene control. Also, as applied to agronomic crops, control of ethylene production may result in increased grain production by slowing the rate of leaf senescence. As such, there are a great many potential applications for the present technique in a wide variety of agricultural crops and products which may be positively effected hereby. Moreover, in some tissues, the application of exogenous ethylene may be used to trigger the typical "autocatylic" effects observed with the natural increase in ethylene production, and thus "override" the decreased endogenous ethylene production for a further positive benefit, such as simultaneous ripening for example.

To provide for a decreased level of ethylene in a plant, a plant cell is transformed with an expression cassette which includes in the 5'-3' direction of transcription, a transcriptional and translation initiation region, a structural gene encoding an ACC deaminase enzyme capable of decreasing levels of precursors of ethylene biosynthesis, and a transcriptional and translational termination regulatory region. The initiation and termination regulatory regions are functional in the host plant cell and may be either homologous or heterologous to the host plant, though either the termination region or the initiation region is heterologous.

As discussed in more detail below, depending upon the plant host and the desired expression profile of interest the regulatory regions will vary. For the most part, the DNA constructs of this invention will provide for regulatory regions functional in plants which are chosen to express in a fairly consistent manner throughout the plant (constitutively) or preferentially (timing and/or tissue specific). In this manner, additional control of ethylene is achieved.

According to the invention, the means of decreasing ethylene concentration is the use of an ACC deaminase. This enzyme affects the availability and/or concentration of ACC, a substrate for ethylene biosynthesis. Considerations for use of a specific ACC deaminase in plant tissue for the metabolism of precursors of ethylene biosynthesis include pH optimum of the enzyme, whether the available substrate is a sole substrate for the enzyme, and co-factors required by the enzyme. The enzyme of interest should have kinetic parameters compatible with the biochemical environment found in the host plant cell. For example, the enzyme may have to compete for substrate with other enzymes. Analysis of the Kₘ and specific activity of the enzymes in question therefore should be considered in determining the suitability of a given enzyme for limiting ethylene production in a given host plant.

The enzyme would thus need to be one which can function under conditions present in the desired target tissue, but otherwise can be any ACC deaminase. In plants, levels of ACC closely correlate with the levels of ethylene production. It is therefore possible to reduce the production of ethylene in the plant tissue of interest, by reducing the levels of ACC in that tissue. Several microorganisms are capable of utilizing ACC, including but not limited to various bacteria which belong to genus *Pseudomonas* and various yeasts such as *Hansenula saturnus.* Of particular interest is the bacterium *Pseudomonas* sp. ACP which uses ACC as a sole nitrogen source. The enzyme is well characterized and converts ACC to ammonia and alpha-ketobutyrate, both common metabolites in plants. The expression of ACC deaminase in plants is designed to scavenge ACC and hence reduce conversion of ACC to ethylene.

ACC deaminase from *Pseudomonas* sp. ACP has a high specificity for ACC (Km = 1.5 mM). ACC deaminase from *H*. *saturnus* has a higher Km value, namely 2.6 mM.

The structural gene for the deaminase may be obtained in a variety of ways. It may be obtained from natural sources such as various prokaryotic cells, including bacteria of the genus *Pseudomonas,* yeasts, and other microorganisms capable of catabolizing ACC.

For the most part, some or all of the deaminase structural gene will be from a natural source or genes at least substantially homologous to such sequences. In some situations it may be desirable to modify all or a portion of the codons, for example to enhance expression, by employing host-preferred codons. Methods for identifying sequences of interest have found extensive exemplification in the literature, although in individual situations, different degrees of difficulty may be encountered. Various techniques include the use of probes where genomic or cDNA libraries may be searched for complementary sequences. Where the structural gene to be inserted is derived from prokaryotic cells, it is desirable to minimize the 3' non-coding region of the prokaryotic gene. The substantial absence of this untranslated region can have a positive effect on the transcription, stability, and/or translation of the mRNA in the plant cells.

The gene may be synthesized in whole or in part, particularly where it is desirable to provide plant preferred codons. Thus, all or a portion of the open reading frame may be synthesized using codons preferred by the plant host. Plant preferred codons may be determined from the codons of highest frequency in the proteins expressed in the largest amount in the particular plant species of interest. Methods for synthesizing sequences and bringing the sequences together are well established in the literature. Where a portion of the open reading frame is synthesized, and a portion is derived from natural sources, the synthesized portion may serve as a bridge between two naturally occurring portions, or may provide a 3'-terminus or a 5'-terminus. Particularly where the signal sequence and the open reading frame encoding the deaminase are derived from different genes, synthetic adapters commonly will be employed. In other instances, polylinkers may be employed, where the various fragments may be inserted at different restriction sites or substituted for a sequence in the pclylinker.

*In vitro* mutagenesis and selection, site-directed mutagenesis, or other means may be employed to obtain mutations of the naturally occurring deaminase gene to produce an enzyme with more desirable physical and kinetic parameters for function in the plant cell, such as a higher affinity for the substrate ACC.

As the deaminase structural gene is other than a plant gene, in order to have expression of the gene in a plant cell transcriptional and translational initiation regulatory regions ("promoters") functional in a plant cell must be provided. Transcription and translation initiation signals functional in plant cells include those from genes which are present in the plant host or other plant species, for example the ribulose bisphosphate carboxylase small subunit transcriptional initiation region, for example from tobacco; those present in viruses such as the cauliflower mosaic virus (CaMV), for example the 35S transcriptional initiation region; and those associated with T-DNA such as the opine synthase transcriptional initiation regions, for example, octopine, mannopine, agropine, etc. The transcription and translation initiation regions may be obtained from the same or different 5' non-coding upstream regulatory region(s). Of particular interest is a transcriptional initiation region in a construct comprising two 35S promoters in tandem (also referred to as a "Double 35S" promoter), mannopine synthase/35S promoter constructs (*See* Comai, *et al., Plant Mol. Biol* (1990) *15*: 373-381), 34S promoters (*See* Sanger, *et al., Plant Mol. Bio.* (1990) *14*:433-443), etc.

Depending upon the application, promoters may obtained from genomic clones of cDNAs which demonstrate preferential expression according to a specific tissue and/or timing profile. For example, petal specific cDNAs (Lawton et al. *Plant Physiol.* (1989) 90:690-696),and cDNAs preferentially expressed in leaves (Dunsmuir et al. *Nucleic Acids Res.* (1983) *11*: 4177-4183), root tips (Pokalsky et al. *Nucleic Acids Res.* (1989) 17:4661-4673), and fruit (Pear et al. Plant Molecular Biology (1989) *13*:639-651) and the like may, be employed. Within such classes of promoters, further differentiation may be observed between more generalized tissue specific promoters and very localized or timing specific promoters. For example, various promoters showing different expression patterns in tomato fruit are described in USPN 4,943,674, issued July 24, 1990 and in WO-A 8 809 334, also EP-A 0 409 629 and EP-A 0 409 625. Depending upon the specific application, one will choose a promoter which will provide the desired expression pattern. Other promoters which are active differently in a specific tissue may be identified by differential screening of a tissue of interest, using methods described for example in USPN 4,943,674 and WO-A 8 809 334.

The regulatory regions may be homologous (derived from the plant host species) or heterologous (derived from source foreign to the plant host species, or synthetic DNA sequence) to the plant host. The term "homologous" includes both indigenous and endogenous sequences. In order to join the promoter(s) to the structural gene, the non-coding 5' region upstream from the structural gene may be removed by endonuclease restriction. Alternatively, where a convenient restriction site is present near the 5' terminus of the structural gene may be restricted and an adapter employed for linking the structural gene to a promoter region, where the adaptor provides for lost nucleotides of the structural gene.

The termination region may be derived from the 3'-region of the gene from which the initiation region was obtained or from a different gene. The termination region may be derived from a plant gene, particularly from a plant gene used to initiate transcription and translation; the tobacco ribulose bisphosphate carboxylase small subunit termination region; a gene associated with the Ti-plasmid such as the octopine synthase termination region; or the *tml* termination region or other 3'-regions known to those skilled in the art.

In developing the expression cassette, the various fragments comprising the regulatory regions and open reading frame may be subjected to different processing conditions, such as ligation, restriction enzyme digestion, resection, *in vitro* mutagenesis, primer repair, use of linkers and adapters, and the like. Thus, nucleotide transitions, transversions, insertions, deletions, or the like, may be performed on the DNA which is employed in the regulatory regions and/or open reading frame. The expression cassette thus may be wholly or partially derived from natural sources, and either wholly or partially derived from sources homologous to the host cell, or heterologous to the host cell. Furthermore, the various DNA constructs (DNA sequences, vectors, plasmids, expression cassettes) of the invention are isolated and/or purified, or synthesized and thus are not "naturally occurring".

During the construction of the expression cassette, the various fragments of the DNA will usually be cloned in an appropriate cloning vector, which allows for amplification of the DNA, modification of the DNA or manipulation by joining or removing of sequences, linkers, or the like. Normally, the vectors will be capable of replication in at least a relatively high copy number in *E. coli.*

A number of vectors are readily available for cloning, including such vectors as pBR322, pUC series, M13 series, etc. The cloning vector will have one or more markers which provide for selection for transformants. The markers will normally provide for resistance to cytotoxic agents such as antibiotics, heavy metals, toxins, or the like. By appropriate restriction of the vector and cassette, and as appropriate, modification of the ends, by chewing back or filling in overhangs, to provide for blunt ends by addition of linkers, by tailing, complementary ends can be provided for ligation and joining of the vector to the expression cassette or component thereof.

After each manipulation of the DNA in the development of the cassette, the plasmid will be cloned and isolated and, as required, the particular cassette component analyzed as to its sequence to ensure that the proper sequence has been obtained. Depending upon the nature of the manipulation, the desired sequence may be excised from the plasmid and introduced into a different vector or the plasmid may be restricted and the expression cassette component manipulated, as appropriate. The manner of transformation of *E*. *coli* with the various DNA constructs (plasmids and viruses) for cloning is not critical to this invention. Conjugation, transduction, transfection or transformation, for example, calcium chloride or phosphate mediated transformation, may be employed.

Depending upon the manner of introduction of the expression construct into the plant, other DNA sequences may be required. Commonly, the expression cassette will be joined to a replication system functional in prokaryotes, particularly *E. coli, so* as to allow for cloning of the expression cassette for isolation, sequencing, analysis, and the like. In most cases the DNA construct will include one or more markers which may allow for selection in the host(s), the markers usually involving biocide resistance, for example antibiotic resistance; heavy metal resistance; toxin resistance; complementation, providing prototrophy to an auxotrophic host; immunity; etc. Often times, markers will be selected which can be detected in a plant host. Where the DNA will be microinjected or propelled into the plant cell, a marker usually will be chosen which allows for direct selection of those cells in which the injected DNA has become integrated and functional.

The use of T-DNA for transformation of plant cells has received extensive study and is amply described in EPA Serial No. 120, 516, Hoekema, *In: The Binary Plant Vector Systems,* Offsetdrukkerij Kanters B.V., Alblasserdam, 1985, Chapter V, Knauf *et al.,* "Genetic Analysis of Host Range Expression by *Agrobacterium",* In: *Molecular Genetics of the Bacteria-Plant Interaction,* Puhler, A. ed., Springer-Verlag, NY, 1983, p. 245, and An *et al., EMBO J.* (1985) *4*:277-284.

Conveniently, explants, cotyledons, or other plant tissue may be cultivated with *A. tumefaciens* or *A. rhizogenes* to allow for transfer of the expression construct to the plant cells, the plant cells dispersed in an appropriate selective medium for selection, grown to callus, shoots grown and plantlets regenerated from the callus by growing in rooting medium. The *Agrobacterium* host will contain a plasmid having the *vir* genes necessary for transfer of the T-DNA to the plant cells and may or may not have T-DNA. If the expression construct is to be inserted into the host cell by injection or electroporation, disarmed Ti-plasmids (lacking the tumor genes, particularly the T-DNA region) may be used.

Various techniques exist for determining whether the desired DNA sequences present in the plant cell are integrated into the genome and are being transcribed. Techniques such as the Northern blot can be employed for detecting messenger RNA which codes for the deaminase. In addition, the presence of expression can be detected in a variety of ways, such as assaying for enzyme activity or immunoassay for the protein product. A desired phenotype in this case is decreased ethylene content in a plant tissue of interest, particularly fruit.

The cells which have been transformed may be grown into plants in accordance with conventional ways. See, for example, McCormick *et al., Plant Cell Reports* (1985) *5*:81-84. These plants may then be grown, and either pollinated with the same transformed strain or different strains, identifying the resulting hybrid having the desired phenotypic characteristic. Two or more generations may be grown to ensure that the subject phenotypic characteristic is stably maintained and inherited and then seeds harvested for use to provide plants having the new phenotypic property expressed in a desired plant tissue, particularly decreased ethylene levels due to metabolism of ethylene synthesis precursors.

The following examples are offered by way of illustration and not by limitation.

### EXPERIMENTAL

### Example 1

### Preparation of Cloned ACC Deaminase Gene Isolation of DNA from Pseudomonas sp. ACP

*Pseudomonas* sp. ACP (Honma and Shimomura *Agric. Biol. Chem.* (1978) *42*:1825-1831) was cultured for 14 hr. at 30°C in a medium composed of 2% glucose, 0.5% bactopeptone and 0.3% yeast extract. Cells were harvested by centrifugation and resuspended in 10X TE buffer (10mM Tris-HCl, pH 7.5, 1 mM EDTA). Sodium dodecyl sulfate and Pronase (Sigma, St. Louis, MO) were then added to 1% and 0.5 mg/ml, respectively. After gentle mixing, the lysis was incubated at 37°C for 30 minutes. DNA was spooled out by overlaying the lysate with 20 µl of ethanol and using a glass rod to spool the DNA from the ethanol/lysate interface. The DNA was dissolved in 10 mM Tris pH 7.5, 1 mM EDTA (TE) at 4°C for extended time. The solution or a portion of it was phenol/chloroform extracted and precipitated at -80°C in 2.5 vol of 100% ethanol. The DNA was peleted by centrifugation and resuspended in TE.

### Construction of Pseudomonas sp. ACP DNA Library

A *Eco*RI/lambda ZapII (Stratagene, La Jolla, CA) library was constructed following the procedures supplied by the manufacturer, from *Pseudomonas* sp. ACP DNA isolated as described above. DNA was completely digested with *Eco*RI which was electrophoresed through a 1.0% agarose gel. The region comprising approximately 3-7 kb in length of the *Pseudomonas* DNA was cut out and electroeluted onto dialysis membrane. The DNA was ethanol precipitated in TE and ligated into the dephosphorylated *Eco*RI site of lambda ZapII (Stratagene, La Jolla, CA) following the manufacturers procedures. The DNA was packaged *in vitro* into viable phage particles. The *Eco*RI lambda ZapII library consists of approximately 5x10⁴ plaque-forming units.

### ACC Deaminase Probe Isolation

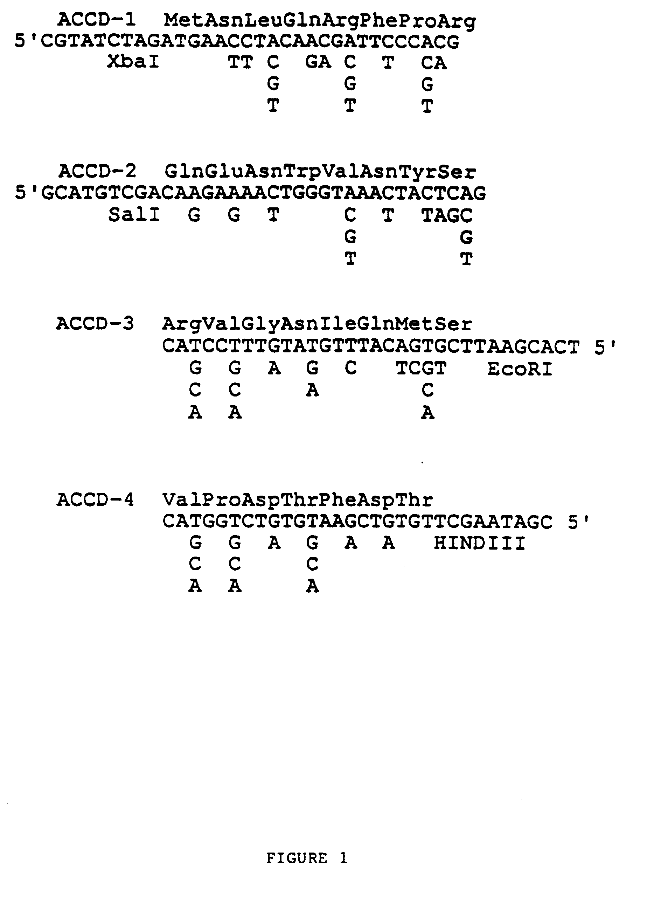

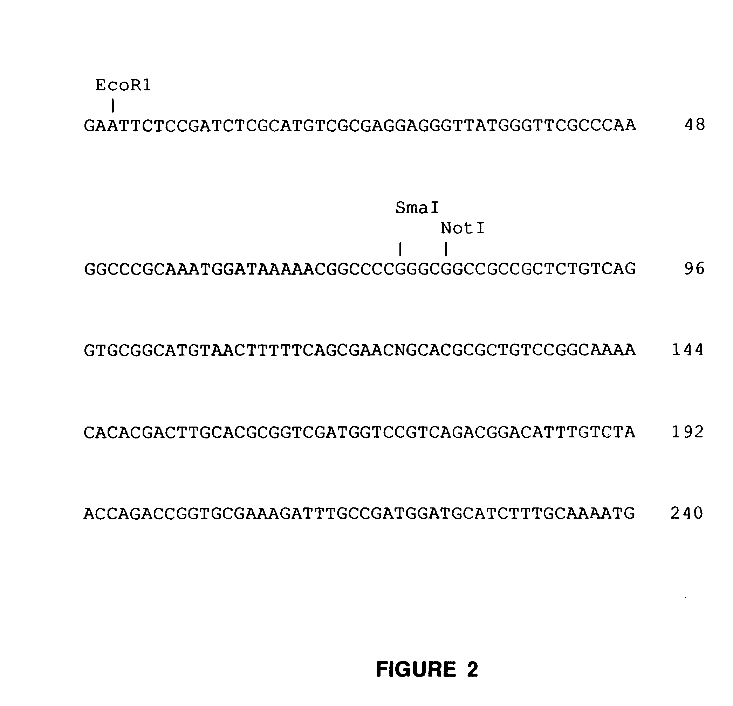

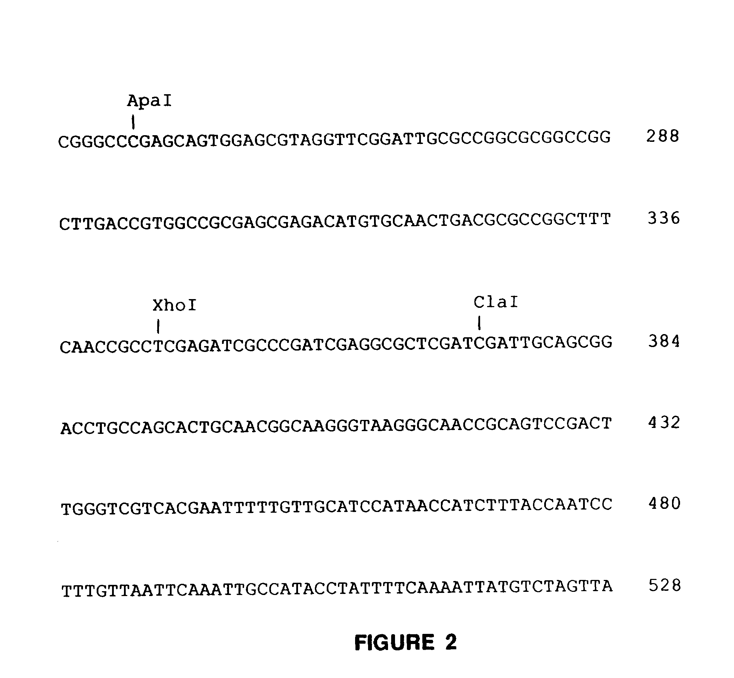

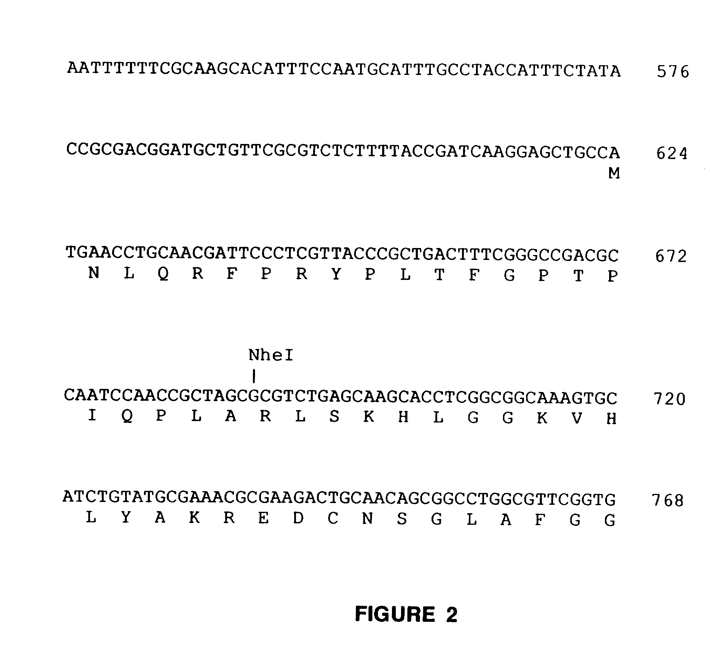

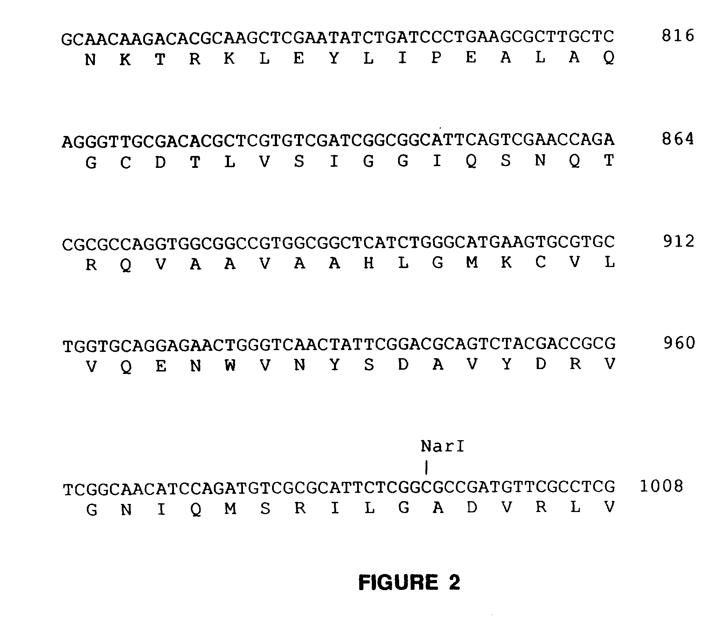

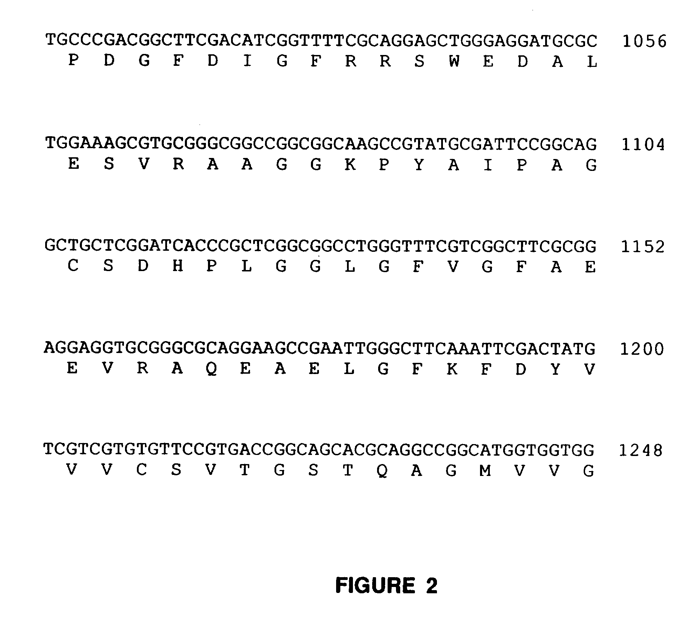

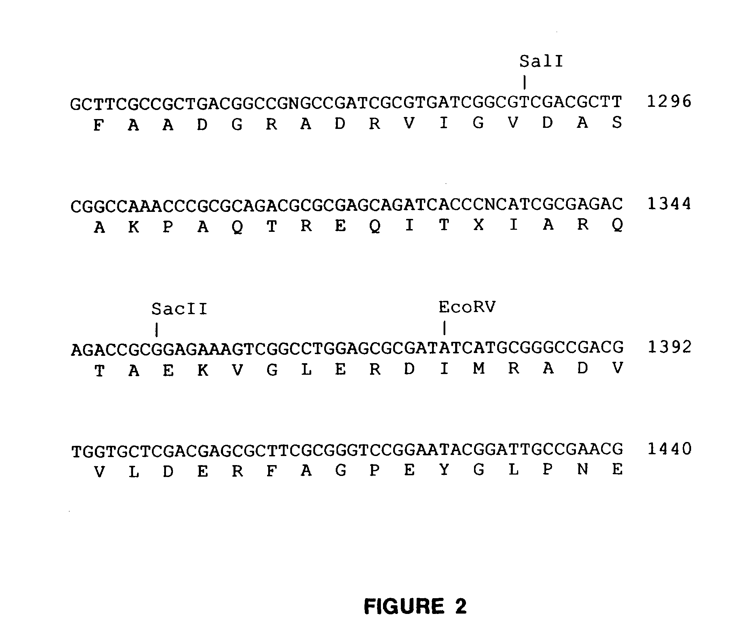

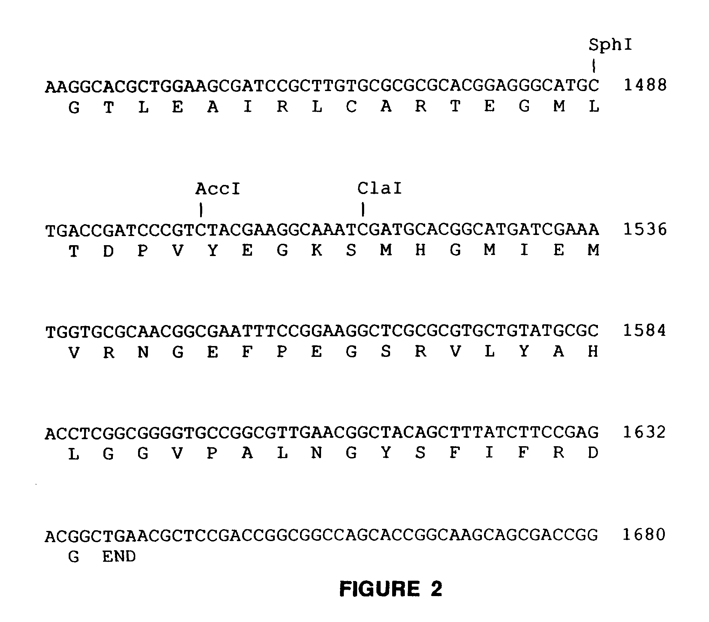

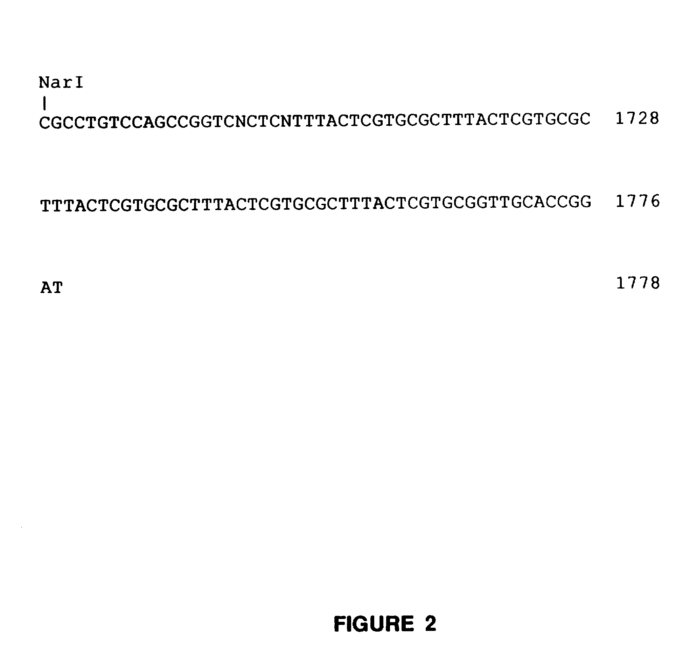

A 369 base pair DNA fragment of the ACC deaminase (ACCD) gene from *Pseudomonas* sp ACP was isolated and labelled with [³²P]dATP and [³²P]dCTP (or [³²P]dCTP) to probe the *Eco*RI/Lambda Zap library for the full-length ACCD gene. Amino acid sequence of peptides (SEQ ID NOS: 1-4) from the N-terminus of ACCD Protein isolated from *Pseudomonas* sp. ACP was provided by Dr. Honma (Hokkaido University) and four synthetic oligonucleotide fragments (SEQ ID NOS: 5-8), see Fig. 1, were synthesized on an Applied Biosystems model 380A DNA synthesizer to sequences that had the least redundancy for use as primers in Polymerase Chain Reactions (PCR) using DNA isolated from *Pseudomonas* sp. as templates. Restriction enzyme sites were synthesized onto the ends of the oligonucleotides for cloning. PCR reactions were run using Taq polymerase (Gene Amp kit) and the DNA thermal cycler (Perkin-Elmer Cetus) using four different combinations of the oligonucleotides as 5'- or 3'- primers. The DNA products were run on a 1% agarose gel and a DNA fragment of approximately 360 base pairs was obtained using the ACCD-1 primer (a 32-mer complementary to the ATG region, plus an *Xba*I site) and the ACCD-3 primer (a 32-mer complementary to a 3'-region sequence, plus an *Eco*RI site) in the PCR reaction. This 360 base pair fragment was then ligated into the *Eco*RI*-Xba*I sites of the Bluescript vector (Stratagene, LaJolla, CA) giving plasmid pCGN2332. The DNA sequence of the ACC deaminase fragment was determined by the dideoxy-chain termination method of Sanger et al. (*Proc. Natl. Acad. Sci. USA* (1977) 74:5463-5467) using the 7-Deaza-dGTP Reagent Kit with Sequenase Version 2 Enzyme (United States Biochemical Corp., Cleveland, OH). The sequence data were analyzed using the IntelliGenetics Suite of molecular programs Gel and SEQ. The sequence (SEQ ID NO: 9) is shown in Fig. 2.

### Library Screening and Plaque Purification

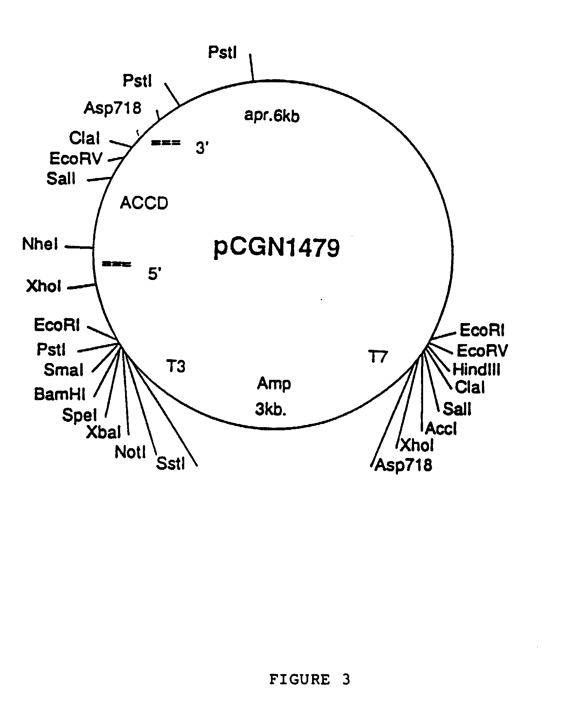

6x10³ *Eco*RI/Lambda Zap II plaque forming units were screened for DNA encoding ACC deaminase by plating the recombinant phage with *E. coli* strain XLI-Blue (Stratagene, La Jolla, CA) on NZY (per liter: 5 g NaCl, 2 g MgCl₂, 10 g NZamine type A (Sheffield Products), 5 g yeast extract and 15 g agar) plates and incubating at 37°C overnight. Plaques were then lifted in duplicate onto nitrocellulose filters, the DNA was denatured (1.5 M NaCl, 0.5M NaOH), neutralized (3M NaCl, 0.5M Tris-HCl, pH 7.5), rinsed in 2 X SCC (Maniatis et al. *Molecular Cloning, a Laboratory Manual* (1982) Cold Spring Harbor, New York) and baked for 1.5 hours at 80°C under vacuum. The filters were then incubated in prehybridization solution (50% formaide, 20X Denhardts, 0.025M NaPO₄, pH 6.5, 5 X SSC, 100 µg/ml denatured salmon sperm DNA, 5mM EDTA and 0.1% SDS) at 42°C for 3 hours. The 369 bp ACCD DNA probe fragment was nick-translated as described by the manufacturer (BRL) using [³²P]dATP and ^{3[2}P]dCTP or [³²P]dCTP alone to a specific activity of ∼10⁸ cpm/µg, boiled, and hybridized to the filters overnight at 42°C in prehybridization solution with 10% dextran sulfate. The filters were then washed four times in 1 X SSC, 0.1% SDS for 15 minutes at 50-55°C. The filters were dried, then exposed to Kodak XAR film at -80°C with an intensifying screen. At least six plaques demonstrated hybridization to the ACCD gene probe after a 5 hr exposure under the above conditions. Clone E11 was picked and reprobed using the radioactive probe. Clone E11 was also rescreened with a polyclonal antibody raised in rabbits against purified ACCD using immunoscreening system (Promega) according to the manufacturers instructions. Clone E11 demonstated a positive reaction against ACCD antibody, and was plaque-purified using the immunoscreening system, then converted to plasmid form in the *E*. *coli* Sure (Stratagene, LaJolla, CA). The resulting plasmid is named pCGN1479. A partial restriction map of pCGN1479 is shown if Fig. 3.

### Sequencing of the ACC deaminase clone

To sequence pCGN1479, the *Cla*I fragment located at the 3' end of the deaminase gene was deleted, resulting in the plasmid ACCD/CLA. ACCD/CLA was digested with *Sac*I and *Eco*RI and ExoIII/S1 deletions were created according to the method of Henikoff *(Meth. Enzymol.* (1987) *155*:156-165). In order to sequence the other strand, ACCD/CLA was digested with *Kpn*I and *Cla*I and ExoIII/S1 deletions were constructed as before. The deletion-containing plasmids were screened using the polymerase chain reaction according to the methods of Sandhu *et al.* (*Biotechniques* (1989) *7*:689-690) to select deletions of appropriate size. Mini-prep DNA (isolated by the method of Maniatis, *et al., supra)* of the selected clones was sequenced using a Sequenase DNA sequencing kit according to the manufacturer's instructions (United States Biochemicals; Cleveland, OH).

### Growth on Minimal Medium with ACC

*E. coli* containing plasmids pCGN1467 and pCGN1458 were tested for the ability to use ACC as the sole nitrogen source. Transformed bacteria were plated on minimal media ( M9 salts-NH₄, 0.2% glucose, 1 mM MgSO₄, 0.1 mM CaCl₂, 25 µg/ml thiamine, and 20 g/l pure agar) containing 0.1% ACC. Growth of *E*. *coli* containing either pCGN1467 and pCGN1468 was observed with or without induction of the *lac* promoter with IPTG, and irrespective of the orientation of the clones to the *lac* promoter.

### Example 2

### Preparation of ACC Deaminase DNA Constructs

### Constitutive Promoter Constructs

An expression cassette, pCGN1431, having a double 35S promoter oriented upstream of a tml 3' transcription termination region and convenient restriction sites therein may be prepared in accordance with as described below. pCGN1431 contains the double cauliflower mosaic virus (CaMV) 35S promoter and the tml 3' region with multiple cloning sites between them. This promoter/terminator cassette is contained in a pUC-derived vector which contains a region that confers resistance to chloramphenicol and is bordered by multiple restriction sites for easy removal.

### Construction of pCGN986

pCGN986 contains a cauliflower mosaic virus 35S (CaMV35) promoter and a T-DNA tml-3' region with multiple restriction sites between them. pCGN986 is derived from another cassette, pCGN206, containing a CaMV35S promoter and a different 3' region, the CaMV region VI 3'-end. The CaMV 35S promoter was cloned as an *Alu*I fragment (bp 7144-7734) (Gardner, *et al., Nucl. Acids Res.* (1981) *9*:2871-2888) into the *Hinc*II site of M13mp7 (Messing, *et al*., *Nucl. Acids Res.* (1981) *9*:309-321) to create C614. An *Eco*RI digest of C614 produced the *Eco*RI fragment from C614 containing the 35S promoter which was cloned into the *Eco*RI site of pUC8 (Vieira and Messing, *Gene* (1982) 19:259-268) to produce pCGN147.

pCGN148a containing a promoter region, selectable marker (kanamycin with 2 ATG's) and 3' region, was prepared by digesting pCGN528 (described below) with *Bgl*II and inserting the *Bam*HI*-Bgl*II promoter fragment from pCGN147. This fragment was cloned into the *Bgl*II site of pCGN528 so that the *Bgl*II site was proximal to the kanamycin gene.

The shuttle vector used for this construct pCGN528, is made as follows: pCGN525 was made by digesting a plasmid containing Tn5 which harbors a kanamycin gene (Jorgensen *et al., Mol. Gen. Genet.* (1979) *177*:65), with *Hind*II-*Bam*HI and inserting the *Hind*III*-Bam*HI fragment containing the kanamycin resistance gene into the *Hind*III*-Bam*HI sites in the tetracycline gene of pACYC184 (Chang and Cohen, *J.* *Bacteriol.* (1978) *134*:1141-1156) pCGN526 was made by inserting the *Bam*HI fragment of 19 of pTiA6 (Thomashow *et al.,* Cell (1980) *19*:729-739) modified with *Xho*I linkers inserted into the *Sma*I site, into the *Bam*HI site of pCGN525. pCGN528 was obtained by deleting the small *Xho*I fragment and religating.

pCGN149a is made by cloning the *Bam*HI kanamycin gene fragment from pMB9KanXXI into the *Bam*HI site of pCGN148a. pMB9KanXXI is a pUC4K variant (Vieira and Messing, *Gene* (1982) *19*:259-268) which is lacking the *Xho*I site, but contains a functional kanamycin gene from Tn903 to allow for efficient selection in *Agrobacterium.*

pCGN149a is digested with *Hind*III and *Bam*HI and ligated to pUC8 digested with *Hind*III and *Bam*HI to produce pCGN169. This removes the Tn903 kanamycin marker. pCGN565 and pCGN169 were both digested with *Hind*III and *Pst*I and ligated to form pCGN203, a plasmid containing the CaMV 35S promoter and part of the 5'-end of the TN5 kanamycin gene (up to the *PstI* site (Jorgensen, *et al., Mol. Gen. Genet.* (1979) *177*:65). A 3' regulatory region was added to pCGN203 from pCGN204 (an *Eco*RI fragment of CaMV (bp 408-6105) containing the region VI 3' cloned into pUC18 (Gardner, et *al., Nucl. Acids Res.* (1981) *9*:2871-2888)) by digestion with *Hind*III and *Pst*I and ligation. The resulting cassette, pCGN206, was the basis for the construction of pCGN986.

The pTiA6 T-DNA tml 3'-sequences are subcloned from the Bam19 T-DNA fragment (Thomashow, *et al.*, *Cell* (1980) 19:729-739) as a *Bam*HI*-Eco*RI fragment (nucleotides 9062 to 12,823, numbering as in (Barker, *et al., Plant Mol. Biol.* (1983) *2*:335-350)) and combined with the pACYC184 (Chang and Cohen, *J. Bacteriol.* (1978) *134*:1141-1156) origin of replication as an *Eco*RI*-Hind*II fragment and a gentamycin resistance marker as a *Bam*HI*-Hind*II fragment to produce pCGN417.

The unique *Sma*I site of pCGN417 (nucleotide 11,207 of the *Bam*19 fragment) was changed to a *Sac*I site using linkers and the *Bam*HI*-Sac*I fragment was subcloned into pCG-N565 to give pCGN971. pCGN565 is a cloning vector based on pUC8-Cm (K. Buckley, Ph.D. Thesis, UC San Diego 1985), but containing the polylinker from pUC18 (Yanish-Perron, *et al., Gene* (1985) *53*:103-119).

The *Bam*HI site of pCGN971 was changed to an *Eco*RI site using linkers to yield pCGN971E. The resulting *Eco*RI*-Sac*I fragment of pCGN971E, containing the tml 3' regulatory sequences, was joined to pCGN206 by digesting with *Eco*RI and *Sac*I to give pCGN975. The small part of the Tn5 kanamycin resistance gene was deleted from the 3'-end of the CaMV 35S promoter by digestion with *Sal*I and *Bgl*II, blunting the ends and ligation with *Sal*I linkers. The final expression cassette pCGN986 contains the CaMV 35S promoter followed by two *Sal*I sites, *Xba*I, *Bam*HI, *Sma*I and, *Kpn*I sites and the tml 3' region (nucleotides 11207-9023 of the T-DNA).

### Construction of pCGN164

The *Alu*I fragment of CaMV (bp 7144-7735) (Gardner, *et al.,* (1981) *supra)* is obtained by digestion with *Alu*I and cloned into the *Hinc*II site of M13mp7 (Vieira and Messing, (1982) *supra*) to create C614. An *Eco*RI digest of C614 produced the *Eco*RI fragment from C614 containing the 35S promoter which was cloned into the *Eco*RI site of pUC8 (Vieira and Messing, (1982) *supra*) to produce pCGN146. To trim the promoter region, the *Bgl*II site (bp7670) was treated with *Bgl*II and *Bal*31 and subsequently a *Bgl*II linker was attached to the *Bal*31 treated DNA to produce pCGN147. pCGN147 was digested with *Eco*RI and *Hph*I and the resultant *Eco*RI*-Hph*I fragment containing the 35S promoter was ligated into *Eco*RI*-Smal*I digested M13mp8 (Vieira and Messing, (1982) *supra*) to create pCGN164.

### Construction of pCGN638

Digestion of CaMV10 (Gardner, *et al., Nucl. Acids Res.* (1981) *9*:2871-2888) with *Bgl*II produced a *Bgl*II fragment containing a 35S promoter region (bp 6493-7670) which was ligated into the *Bam*HI site of pUC19 (Norrander, *et al., Gene* (1983) *26*:101-106) to create pCGN638.

### Construction of pCGN2113

pCGN164 was digested with *Eco*RV and *Bam*HI to release a *Eco*RV-*Bam*HI fragment which contained a portion of the 35S promoter (bp 7340-7433); pCGN638 was digested with *Hind*III and *Eco*RV to release a *Hind*III-*Eco*RV fragment containing a different portion of the 35S promoter (bp 6493-7340). These two fragments were ligated into pCGN986 which had been digested with *Hind*III and *Bam*HI to remove the *Hind*III-*Bam*HI fragment containing the 35S promoter; this ligation produced pCGN639, which contained the backbone and tml-3' region from pCGN986 and the two 35S promoter fragments from pCGN164 and pCGN638. pCGN638 was digested with *Eco*RV and *Dde*I to release a fragment of the 35S promoter (bp 7070-7340); the fragment was treated with the Klenow fragment of DNA polymerase I to create blunt ends, and was ligated into the *Eco*RV site of pCGN639 to produce pCGN2113 having the fragment in the proper orientation.

### Construction of pCGN1431

The *Sal*I*-Eco*RI fragment of pCGN2113, which contained the entire promoter-polylinker-3' cassette, was removed by *Sal*I*-Eco*RI digestion and cloned into *Sal*I*-Eco*EI digested pCGN565 to create pCGN2120. pCGN2120 was digested to completion with *Pst*I and then religated. A clone was selected which had deleted only the 858 bp *Pst*I-*Pst*I fragment (9207-10065, Barker, *et al.,* (1983) *supra*) from the tml 3' region to create pCGN1431.

In order to prepare convenient restriction sites for insertion of the ACC deaminase gene into pCGN1431, oligonucleotides which are complimentary to the 5' and 3' ends of the ACC deaminase gene are synthesized on an Applied Biosystems model 380A DNA synthesizer (Applied Biosystems; Foster City, CA). The first oligonucleotide is complementary to bp 692 to 672 of the deaminase sequence, as shown in Fig. 2, and, in addition, has a *Bam*HI site at the 5' end. The second oligonucleotide comprises sequence that is complementary to bp 1615 to 1635 of the ACC deaminase structural gene, and, in addition, has a *Sst*I restriction site at the 5' end. When a PCR reaction is carried out, according to the manufacturer's instructions (Perkin Elmer Cetus; Emeryville, CA), using the above oligonucleotides as primers and DNA containing the ACC deaminase gene as template, the PCR product will be a fragment containing a *Bam*HI site, the ACC deaminase coding region, and a SstI site. This fragment may conveniently be digested with *Bam*HI and *Sst*I, and cloned into *Bam*HI*-Sst*I-digested pCGN1431.

### Fruit Specific Promoter DNA Constructs

An expression cassette, pCGN1240, having a 2A11 promoter oriented upstream of a 2A11 3' transcription termination region, with convenient restriction sites between may be prepared in accordance with the teachings in EP-A-0 409 625 pCGN1240 contains 3.8 kb of 2A11 5' non-coding sequence and 2 kb of 2A11 3' non-coding sequence, with multiple cloning sites between them. The cassette is flanked by restriction sites for the enzymes *Kpn*I, *Apa*I, *Dra*II, *Xho*I, *Sal*I, *Hind*III, and *Eco*RI at the 5' end and *Eco*RI, *Eag*I, *Not*I, *Sac*II, *Bst*XI, and *Sac*I at the 3' end. The backbone contains a region that confers resistance to chloramphenicol.

In order to prepare convenient restriction sites for insertion of the ACC deaminase gene into pCGN1240, oligonucleotides which are complimentary to the 5' and 3' ends of the ACC deaminase gene are synthesized on an Applied Biosystems model 380A DNA synthesizer (Applied Biosystems; Foster City, CA). The first oligonucleotide is complementary to bp 692 to 672 of the deaminase sequence (SEQ ID NO: 9), as shown in Fig. 2, and, in addition, has a *Bam*HI site at the 5' end. The second oligonucleotide comprises sequence that is complementary to bp 1615 to 1635 of the ACC deaminase structural gene, and, in addition, has a *Pst*I restriction site at the 5' end. When a PCR reaction is carried out according to the manufacturer's instructions (Perkin Elmer Cetus; Emeryville, CA) using the above oligonucleotides as primers and DNA containing the ACC deaminase gene as template, the PCR product will be a fragment containing a *Bam*HI site, the ACC deaminase coding region, and a *Pst*I site. This fragment may conveniently be digested with *Bam*HI and *Sst*I*,* and cloned into *Bam*HI*-Pst*I-digested pCGN1240.

### Construction of Binary Vectors

When the respective expression cassette has been constructed, it may be placed into a convenient binary vector, such as pCGN1547 (see below). For example, the 35S CaMV constitutive promoter cassette described above is digested with *Pst*I, and the fragment containing the double 35S-5'/ACC deaminase/tml-3' region is ligated with *Pst*I-digested pCGN1547. And, as an additional example, the fruit-specific promoter cassette described above is digested with *EcoRI,* and the fragment containing the 2A11-5'/ACC deaminase/2A11-3' is ligated with *Eco*RI-digested pCGN1547. These ligations will result in binary vectors suitable for introduction into *Agrobacterium tumefaciens.* pCGN1547 (McBride and Summerfelt, *Plant Mol. Biol.* (1990) *14(27)* :269-276) is a binary plant transformation vector containing the left and right T-DNA borders of *Agrobacterium tumefaciens* octopine Ti-plasmid pTiA6 (Currier and Nester, *J. Bact.* (1976) *126*:157-165), the gentamicin resistance gene of pPh1JI (Hirsch and Beringer, *Plasmid* (1984) *9*:2871-2890), and *Agrobacterium rhizogenes* Ri plasmid origin of replication from pLJbB11 (Jouanin et al., *Mol. Gen. Genet.* (1985) *201:*370-374)*,* the *mas* promoter region and *mas* 3' region of pTiA6 with the kanamycin resistance gene of Tn5 (Jorgensen *et al., Mol. Gen Genet.* (1979) *177*:65), a ColE1 origin of replication from pBR322 (Bolivar *et al., Gene* (1971) *2*:95-133), and a *lac*Z' screenable marker gene from pUC18 (Norrander et *al., Gene* (1983) *26*:101-106).

### Introduction of Binary Vectors into Agrobacterium tumefaciens

The binary vectors may be introduced into *Agrobacterium tumefaciens* strain LBA4404 (Horsch *et al., Science* (1985) *227*:1229-1231) using the method of Holsters *et al., Mol.* Gen. *Genet.* (1978) *163*:181-187. Subsequently, the transformed *Agrobacterium tumefaciens* may be used to infect suitable host plant tissue from which transgenic plants may be regenerated.

### Example 3

### Petunia Transformation Protocol

Feeder plates are prepared which contain solid media with MS salts (Murashige and Skoog, *Physiol. Plant.* (1962) *15*:473-498), B5 vitamins (Gamborg, *et al., Exp. Cell. Res.* (1968) *50*:148-151), 0.7% phytagar, 3% sucrose, 0.1 mg/L naphthalene acetic acid, and 1.0 mg/L benzyladenine on the surface of which is spread 0.5 mL of 7 day old tobacco suspension cells. The plates are bagged and placed under low light intensity (roughly 30 µEm⁻²S⁻¹). Approximately 24 hours later, a sterile #1 Whatman filter paper (Whatman Ltd., Maidstone, England) is placed on top of the feeder plate medium. Leaves of 4-12 week old Mitchell *Petunia* plants, which are grown in the Growth Chamber at 25°C, 16 hours light/8 hours dark, 215 uEm⁻²S⁻¹, are collected, transported to the lab in water, and surface sterilized by a 45 second dip in 70% ethanol, followed by a 45 second dip in 50% bleach (v/v) and 3 sterile water rinses. The sterilized leaves are then cut into explants roughly 5-10mm by 5-10mm long, plated on feeder plates with filters, and incubated for 24 hours in the dark or at low light intensity at 24°C.

The *Agrobacterium* are grown for 4-5 days on solid AB minimal medium (Watson, *et al., J. Bacteriol.* (1975) *123*:255-264) containing 100 mg/L gentamycin sulfate and 100 mg/L streptomycin sulfate. Single colonies are inoculated into 5 mls of MG/L broth (50% Luria broth and 50% mannitolglutamate salts medium (Garfinkel and Nester, *J. Bacteriol.* (1980) *144*:732-743)) and are incubated overnight on a shaker at 30°C and 180 R.P.M. before cocultivation.

Following the pre-incubation period, the explants are dipped into the bacterial suspension of 5.0 x 10⁸ cells/mL for approximately 3 minutes, blotted on sterile paper towels, and replated on the same plates. After 48 hours, the explants are placed on selection medium containing the same plate medium as above (but without feeder cells and filter) plus 300 mg/L kanamycin and either 350 mg/l cefotaxime or 500 mg/L carbenicillin. The explants are transferred to fresh media every 2 weeks; at the 2 week transfer and thereafter, the kanamycin level of the medium is dropped from 300 to 100 mg/L. Shoots are harvested beginning about 4 weeks after co-cultivation and placed on plates of rooting medium containing MS salts, B5 vitamins, 0.7% bactoagar, 3% sucrose, 1 mg/L indole-3-butyric acid, 100 mg/L kanamycin, and 200 mg/L cefotaxime or 350 mg/l carbenicillin. Shoots rooted in approximately 2 weeks and are then transplanted into soil and placed in growth chambers. All *in vitro* tissue is grown at 24-28°C, 16 hours light, 8 hours dart, light intensity 80-100 uEm⁻²S⁻¹.

Plants in the growth chamber flowered in 6-8 weeks and set seed 8-12 weeks after flowers are hand-pollinated; seed is obtained from transgenic plants 14-18 weeks after co-cultivations are initiated.

### Example 4

### ACC Deaminase in Plants

The ACC deaminase gene from *Pseudomonas* sp. ACP has been used to construct plasmid vectors for the expression of ACC deaminase activity in plants. ACC deaminase was detected immunologically in electroporated tobacco protoplasts and in transformed petunia leaves. There was an indication of ACC deaminase activity in extracts of transformed plant tissue. Tissue from one of the transformants appeared less able to convert ACC to ethylene in an *in vitro* assay.

### Expression Vectors

An expression cassette, pCGN2187 was created by removal of the *Eco*RV-*Bam*HI fragment of pCGN1431 (See, Example 2) and replacement with the *Eco*RV*-Bam*HI fragment of pCGN986 (*See,* Example 2) to produce a "double" 35S promoter with 112 bp of untranslated leader sequence relative to the mRNA cap site instead of the 2 bp present in pCGN1431. A *Bam*HI*-Sac*I fragment of the ACC deaminase gene (prepared by PCR as described for insertion into pCGN1431) was then inserted into pCGN2187 to create pCGN1492. pCGN1492 was electroporated into tobacco protoplasts as described below. A *Pst*I fragment from pCGN1492 was inserted into pCGN1547 to create the binary vector pCGN1493. pCGN1493 was used to transform petunia as described in Example 3.

### Electroporation Data

Protoplast donor plants of *Nicotiana tabaccum* (cv. Xanthi) were grown in glass jars under aseptic conditions as described by Facciotti and Pilet (Facciotti, D. and Pilet, P.E. *Pl. Sci. Let.* (1979) 15,1). Apical shoots were placed into 100ml of a 0.7% phytagar medium containing MS salts plus 30g/l sucrose, 1.0mg/l IRA and 0.15mg/l kinetin, pH 5.55. The *Xanthi* cultures were grown at 23 + or - 3°C under a 12 hour dark/light regime.

Young leaves were selected from 3-4 week old plants during the dark portion of the cycle. Leaf sections were vacuum infiltrated to 300 militorr with a 6% sorbitol solution containing 0.04% pectinase (Pectolyase Y-23, Seishin Pharmaceutical Co., Ltd., Japan) 0.45% cellulase (Onozuka RS, Yakult Pharmaceutical Industry Co., Japan) and 0.5% potassium dextran sulfate, pH to 5.55.

The plant material was digested for 3-4 hours while gently shaking at 50 rpm. Protoplasts were separated from debris by passing macerate through a 52um mesh nylon screen. Protoplasts were pelleted by centrifugation at 150 X g for 5 minutes and washed 3 times using a 7% sorbitol solution containing 1mm CaCl and 10mM hepes. Protoplasts were suspended at a density of 2-3X10⁶ in an electroporation buffer containing 6% sorbitol, 10mM hepes, 140mM NaCl, 5mM CaCl, pH 7.1 and a carrier DNA (pUC19 or salmon sperm DNA) concentration of 175ug/ml.

Electroporation was carried out in electroporation cuvetts as described by Potter et al., (Potter, H., Weir, L. and Leder, P., *Proc. Natl. Acad. Sci. USA* (1984) *81*:7161-7165). The appropriate plasmid DNA was added to lml aliquots of the protoplasts in electroporation buffer and then transferred to electroporation cuvetts where a single pulse of 600V/cm was discharged from a 1250 uf capacitor. Following electroporation, protoplasts were transferred to 9ml of a medium containing MS salts plus 30g/l sucrose, 0.6mg/l NRA, 0.2mg/1 2,4-D, 0.8mg/l kinetin and 5.5% sorbitol and allowed to incubate at 25°C for 48 hours without light. Samples were then harvested by centrifugation at 50 X g for 8 minutes. The supernatant was discarded and pellets were frozen in liquid nitrogen and stored at -70°C until assayed.

### Western Blots

Samples of electroporated tobacco cells or transformed petunia leaf cells were frozen in liquid nitrogen and ground while frozen. Sample buffer (2X, Laemmli, *Nature* (1970) *227*:680-685) was heated to 100°C and added to ground samples, followed by incubation in a boiling water bath for at least 5 minutes. Samples were loaded onto a 10% polyacrylamide gel and resolved by electrophoresis (Laemmli). Proteins were electroblotted onto nitrocellulose and reacted with ACCD antibodies (See, Example 1). ACCD was identified by an alkaline phosphatase immunoscreening system as described by the vendor (Promega; Madison, WI).

Electroporation of pCGN1492 into tobacco protoplasts resulted in a protein band of the size expected for ACCD (approximately 36kDa) as visualized by immunodetection. A protein band corresponding to ACCD was also detected in pCGN1493 transformed petunia leaf extracts, including transformant 1493-11.

### Enzyme Assay

Transformed petunia tissue was homogenized with 4 volumes of 100mM Bis-Tris, pH 6.5, 5mM EDTA, 10mM BME and 10uM leupeptin containing 0.5gm/gm plant tissue of insoluble polyvinyl polypyrrolidone ("PVPP") in a ground glass homogenizer. The PVPP and insoluble plant material was removed by centrifugation in a microfuge. Powdered ammonium sulfate (0.137gm/ml of supernatant) was mixed into the solution, which was then incubated on ice for 1.5 hr. The precipitated material was removed by centrifugation and additional ammonium sulfate (0.17gm/ml supernatant) was added followed by incubation overnight on ice. The pellet was recovered by centrifugation and resuspended in 150ul of 100mM KPO₄, pH 7.5, 1mm EDTA, lmM BME and 10uM leupeptin and dialyzed against the same buffer without leupeptin.

ACC deaminase activity was measured by the accumulation of alpha-ketobutyrate from ACC. Determination of alpha-ketobutyrate utilized the method of H. Katsuki, T. Yoshida, C. Tanegashima and S. Tanka, (*Anal. Biochem.* (1971) *43*:349-356) modified as follows: Reactions contain 100mM Tris pH 8.5, 50mM ACC and protein extract in a final volume of 0.1-0.2ml. Following incubation at 30°C, an equal volume of saturated dinitrophenylhydrazine (DNPH) in methanol and 10ul of concentrated HCl per 0.lml DNPH was added and incubation was continued at 50°C for 30 minutes. Four ml of 10% KOH in 80% methanol per 0.2ml volume was added and the absorbance at 480 nm was determined as a measure of alpha-ketobutyrate concentrations.

Alternatively, ACC deaminase activity may be detected according to the following method.

Transformed petunia leaf tissue was frozen in liquid nitrogen, ground to a powder and extracted with an equal volume of 200mM Tris, pH 8.5, 2mM EDTA, 1.5% insoluble PVPP, lmM β-mercaptoethanol, 2ug/ml leupeptin, 200uM phenylmethylsulfonylfluroide (PMSF) and 6.8mg/ml diethyldithiocarbamate. The insoluble material was removed by centrifugation, and 98ul of cleared extract was added to a final mixture of 50nM 1-aminocyclopropane-1-carboxylate (ACC) containing 8-16nCi of [¹⁴C]ACC. The reaction was incubated at 30°C for 1 hour and then passed over a column of Dowex-50 W (hydrogen form) which absorbs ACC but not α-ketobutyrate. The column was washed with 100ul of 10mM Tris, pH 8.0 and the eluate and wash pooled and radioactively measured using a liquid scintillation counter. Activity was measured as the amount of radioactive α-ketobutyrate formed in the reaction and released from the column.

Leaf discs were excised using a cork borer, weighed and placed in a 4ml vial with 200ul water. The samples were placed in a vacuum desiccator and a vacuum was applied for 5 minutes. The vials were then sealed with caps containing rubber septa. Samples were removed with a syringe after extended incubation at room temperature and injected into a flame ionization gas chromatograph to measure ethylene.

Evidence for ACC deaminase activity was seen in extracts from 1493-11 but not in the control plant tissue transformed with a gus marker gene.

### Ethylene Production

Leaf samples from transformed petunia plants were incubated in a solution of 10mM Tris, pH 8.0, 10uM ACC in 4ml vials sealed with a neoprene septum. Following incubation, 1ml gas samples were injected into a gas chromatograph flame ionization detector system equipped with an alumina column. The ethylene peak was identified and quantified by injection a series of ethylene standards. Leaf tissue from 1493-11 appeared less able to convert ACC to ethylene as compared to control plant tissue.

The above results demonstrate the ability to identify DNA sequences encoding ACCD, to isolate the sequences and manipulate them, and to transfer the sequences, and as a consequence, the ability to metabolize ACC, from *Pseudomonas* to *E. coli.* From these sequences, expression cassettes can be produced which will provide for expression of an enzyme such as ACCD which metabolizes an ethylene biosynthesis precursor in a plant tissue cf interest. Thus, the ethylene concentration of a particular plant part may be modified by including in the expression cassette inducible regulatory sequences which allow for differentiated cell production of in enzyme such as ACC deaminase. Thus, the ethylene concentration of a particular plant part may be modified without adverse effects on the plant. The effected plant part remains capable of responding to the effects of ethylene at a later time, particularly upon application of exogenous ethylene.

All publications and patent applications mentioned in this specification are indicative of the level of skill cf those skilled in the art to which this invention pertains.

## Claims (Claims for the following Contracting State(s): AT, BE, CH, LI, DE, DK, FR, GB, GR, IT, LU, NL, SE)

1. A DNA construct comprising:
in the 5'-3' direction of transcription, the following components, a transcriptional and translational initiation region functional in a plant cell, a DNA sequence encoding a 1-aminocyclopropane-1-carboxylic acid (ACC) deaminase, which deaminase is capable of metabolising 1-aminocyclopropane-1-carboxylic acid (ACC) and thus reducing ethylene biosynthesis, and a transcriptional termination region, wherein said components are operably linked and wherein at least one of said initiation and termination regions is other than the region naturally associated with said DNA sequence.

2. The DNA construct according to claim 1 wherein the DNA sequence encodes a bacterial 1-aminocyclopropane-1-carboxylic acid (ACC) deaminase.

3. The DNA construct according to claim 2, wherein said DNA sequence encoding said ACC deaminase is obtainable from *Pseudomonas sp.* ACP.

4. The DNA construct according to claim 3, wherein said DNA sequence encodes *Pseudomonas sp.* ACP ACC deaminase as shown in Figure 2.

5. The DNA construct according to any one of the preceding claims further comprising a broad spectrum prokaryotic replication system.

6. The DNA construct according to any one of the preceding claims further comprising a region of homology of at least the right T-DNA border of a size sufficient to provide for integration into a plant genome, said region being joined to said construct.

7. The DNA construct according to any one of the preceding claims, wherein said transcriptional and translational initiation region comprises a double 35S CaMV promoter.

8. The DNA construct according to any one of claims 1 to 6, wherein the translational initiation region comprises a petal-specific, leaf-specific, root tip-specific or fruit-specific promoter.

9. A method for reducing ethylene levels in plant tissue comprising: transforming a plant cell with a DNA sequence encoding 1-aminocyclopropane carboxylic acid (ACC) deaminase, thereby to obtain plant tissue comprising one or more plant cells which comprise a DNA sequence encoding a 1-aminocyclopropane-1-carboxylic acid (ACC) deaminase, which deaminase is capable of metabolizing -aminocyclopropane--carboxylic acid (ACC) and thus reducing ethylene biosynthesis; and growing said tissue under conditions whereby said gene is expressed and the ethylene level of said plant tissue is reduced compared to the level normally present in said plant tissue.

10. The method of claim 9 wherein the DNA sequence is comprised in a construct according to any one of claims 1 to 8.

11. The method of claim 10 wherein said enzyme is obtainable from *Pseudomonas sp.* ACP.

12. The method of any one of claims 9 to 11 wherein said DNA sequence is under the transcriptional and translational initiation control of petal-specific, leaf-specific, root tip-specific or fruit-specific promoter.

13. A plant cell comprising a DNA construct according to any one of claims 1 to 8.

14. A plant cell comprising a bacterial 1-aminocyclopropane-l-carboxylic acid (ACC) deaminase.

15. A method of producing a 1-aminocyclopropane carboxylic acid (ACC) deaminase, which deaminase is capable of metabolizing 1-aminocyclopropane-1-carboxylic acid (ACC), and thus reducing the level of ethylene present in a host cell comprising:
transforming a host cell with a DNA construct according to any one of claims 1 to 8 and growing said cell under conditions which will permit the production of said deaminase.

16. The method of claim 15 wherein said host cell is a plant cell and said construct is integrated into the genome of said plant cell.

17. The method of claim 16 wherein said plant cell is *in vivo.*

18. A plant cell comprising an enzyme as defined in claim 15.

19. A plant cell having a reduced ethylene level compared to the level normally present in said cell obtainable by the method of any one of claims 9 to 12 or 16.

20. A method for altering ethylene levels in a plant comprising:
transforming a plant cell with a DNA sequence encoding 1-aminocyclopropane carboxylic acid (ACC) deaminase; obtaining a plant comprising one or more cells comprising said DNA sequence; and expressing said DNA sequence in said plant, whereby expression of said DNA sequence reduces the ACC available for ethylene conversion in said plant.

21. The method of claim 20 wherein the DNA sequence is comprised within a construct according to any one of claims 1 to 8.

## Claims (Claims for the following Contracting State(s): ES)

1. A method for reducing ethylene levels in plant tissue comprising: transforming a plant cell with a DNA sequence encoding 1-aminocyclopropane carboxylic acid (ACC) deaminase, thereby to obtain plant tissue comprising one or more plant cells which comprise a DNA sequence encoding a 1-aminocyclopropane-1-carboxylic acid (ACC) deaminase, which deaminase is capable of metabolizing 1-aminocyclopropane-1-carboxylic acid (ACC) and thus reducing ethylene biosynthesis; and growing said tissue under conditions whereby said gene is expressed and the ethylene level of said plant tissue is reduced compared to the level normally present in said plant tissue.

2. The method of claim 1 wherein the DNA sequence is comprised in a DNA construct comprising:
in the 5'-3' direction of transcription, the following components, a transcriptional and translational initiation region functional in a plant cell, a DNA sequence encoding a 1-aminocyclopropane-1-carboxylic acid (ACC) deaminase, which deaminase is capable of metabolising 1-aminocyclopropane-1-carboxylic acid (ACC) and thus reducing ethylene biosynthesis, and a transcriptional termination region, wherein said components are operably linked and wherein at least one of said initiation and termination regions is other than the region naturally associated with said DNA sequence.

3. The method according to claim 2 wherein the DNA sequence encodes a bacterial 1-aminocyclopropane-1-carboxylic acid (ACC) deaminase.

4. The method according to claim 3, wherein said DNA sequence encoding said ACC deaminase is obtainable from *Pseudomonas sp.* ACP.

5. The method according to claim 4, wherein said DNA sequence encodes *Pseudomonas sp.* ACP ACC deaminase as shown in Figure 2.

6. The method according to any one of claims 2 to 5, wherein the construct further comprises a broad spectrum prokaryotic replication system.

7. The method according to any one of claims 2 to 6 wherein the construct further comprises a region of homology of at least the right T-DNA border of a size sufficient to provide for integration into a plant genome, said region being joined to said construct.

8. The method according to any one of claims 2 to 7, wherein said transcriptional and translational initiation region comprises a double 35S CaMV promoter.

9. The method according to any one of claims 2 to 7, wherein the translational initiation region comprises a petal-specific, leaf-specific, root tip-specific or fruit-specific promoter.

10. A method of producing a 1-aminocyclopropane carboxylic acid (ACC) deaminase, which deaminase is capable of metabolizing 1-aminocyclopropane-1-carboxylic acid (ACC), and thus reducing the level of ethylene present in a host cell comprising:
transforming a host cell with a DNA construct as defined in any one of claims 2 to 9 and growing said cell under conditions which will permit the production of said deaminase.

11. The method of claim 10 wherein said host cell is a plant cell and said construct is integrated into the genome of said plant cell.

12. The method of claim 11 wherein said plant cell is *in vivo.*

13. A method for altering ethylene levels in a plant comprising:
transforming a plant cell with a DNA sequence encoding 1-aminocyclopropane carboxylic acid (ACC) deaminase; obtaining a plant comprising one or more cells comprising said DNA sequence; and expressing said DNA sequence in said plant, whereby expression of said DNA sequence reduces the ACC available for ethylene conversion in said plant.

14. The method of claim 13 wherein the DNA sequence is comprised within a construct as defined in any one of claims 2 to 9.

15. A DNA construct comprising:
in the 5'-3' direction of transcription, the following components, a transcriptional and translational initiation region, a DNA sequence encoding a 1-aminocyclopropane-1-carboxylic acid (ACC) deaminase, which deaminase is capable of metabolising 1-aminocyclopropane-1-carboxylic acid (ACC) and thus reducing ethylene biosynthesis and a transcriptional termination region, wherein said components are operably linked and wherein at least one of said initiation and termination regions is other than the region naturally associated with said DNA sequence.

16. The DNA construct according to claim 15, wherein the DNA sequence encodes a bacterial 1-aminocyclopropane-1-carboxylic acid (ACC) deaminase.

17. The DNA construct according to claim 16, wherein siad DNA sequence encoding said ACC deaminase is obtainable from *Pseudomonas sp.* ACP.

18. The DNA construct according to claim 17, wherein said DNA sequence encodes *Pseudomonas sp.* ACP ACC deaminase as shown in Figure 2.

19. The DNA construct according to any one of claims 15 to 18 further comprising a broad spectrum prokaryotic replication system.

20. The DNA construct according to any one of claims 15 to 19 further comprising a region of homology of at least the right T-DNA border of a size sufficient to provide for integration into a plant genome, said region being joined to said construct.

21. The DNA construct according to any one of claims 15 to 20, wherein said transcriptional and translational initiation region comprises a double 35S CaMV promoter.

22. The DNA construct according to any one of claims 15 to 21, wherein the translational initiation region comprises a petal-specific, leaf-specific, root tip-specific or fruit-specific promoter.

23. A plant cell comprising a DNA construct according to any one of claims 15 to 22.

24. A plant cell comprising a bacterial 1-aminocyclopropane-1-carboxylic acid (ACC) deaminase.

25. A plant cell comprising a 1-aminocyclopropane carboxylic acid (ACC) deaminase, which deaminase is capable of metabolizing 1-aminocyclopropane-1-carboxylic acid (ACC), and thus reducing the level of ethylene present in a host cell.

26. A plant cell having a reduced ethylene level compared to the level normally present in said cell obtainable by the method of any one of the claims 1 to 9 or 11.

## Patentansprüche (Patentansprüche für folgende(n) Vertragsstaat(en): AT, BE, CH, LI, DE, DK, FR, GB, GR, IT, LU, NL, SE)

1. DNA-Konstrukt, umfassend
in der 5'-3'-Richtung der Transkription die folgenden Komponenten: einen in einer Pflanzenzelle funktionellen Transkriptions- und Translationsinitiationsbereich, eine eine 1-Aminocyclopropan-1-carbonsäure(ACC)-desaminase kodierende DNA-Sequenz, wobei die Desaminase in der Lage ist, 1-Aminocyclopropan-1-carbonsäure (ACC) zu metabolisieren und somit die Ethylenbiosynthese zu verringern, und einen Transkriptionsterminationsbereich, wobei die Komponenten in operabler oder funktioneller Weise verknüpft sind und wobei mindestens einer der Initiations- und Terminationsbereiche nicht der natürlicherweise mit der DNA-Sequenz assoziierte Bereich ist.

2. DNA-Konstrukt nach Anspruch 1, wobei die DNA-Sequenz eine bakterielle 1-Aminocyclopropan-1-carbonsäure (ACC)-desaminase kodiert.

3. DNA-Konstrukt nach Anspruch 2, wobei die die ACC-Desaminase kodierende DNA-Sequenz aus *Pseudomonas sp.* ACP erhalten werden kann.

4. DNA-Konstrukt nach Anspruch 3, wobei die DNA-Sequenz *Pseudomonas sp.* ACP ACC-Desaminase, wie in Figur 2 gezeigt, kodiert.

5. DNA-Konstrukt nach einem der vorangegangenen Ansprüche, das ferner ein prokaryotisches Breitspektrumreplikationssystem umfaßt.

6. DNA-Konstrukt nach einem der vorangegangenen Ansprüche, das ferner einen Bereich von Homologie an mindestens dem rechten T-DNA-Rand mit einer ausreichenden Größe umfaßt, um eine Integration in ein Pflanzengenom zu ermöglichen, wobei der Bereich mit dem Konstrukt verknüpft ist.

7. DNA-Konstrukt nach einem der vorangegangenen Ansprüche, wobei der Transkriptions- und Translationsinitiationsbereich einen doppelten 35S-CaMV-Promotor umfaßt.

8. DNA-Konstrukt nach einem der Ansprüche 1 bis 6, wobei der Translationsinitiationsbereich einen Kronblatt-spezifischen, Laubblatt-spezifischen, Wurzelspitzen-spezifischen oder Frucht-spezifischen Promotor umfaßt.

9. Verfahren zum Verringern von Ethylenkonzentrationen in Pflanzengewebe, welches umfaßt: eine Pflanzenzelle mit einer 1-Aminocyclopropancarbonsäure (ACC) -desaminase kodierenden DNA-Sequenz zu transformieren, um dadurch Pflanzengewebe zu erhalten, das eine oder mehrere Pflanzenzellen umfaßt, die eine eine 1-Aminocyclopropan-1-carbonsäure (ACC)-desaminase kodierende DNA-Sequenz umfaßt bzw. umfassen, wobei die Desaminase in der Lage ist, 1-Aminocyclopropan-1-carbonsäure (ACC) zu metabolisieren und somit die Ethylenbiosynthese zu verringern, und das Gewebe unter Bedingungen zu züchten, wodurch das Gen exprimiert wird und die Ethylenkonzentration des Pflanzengewebes im Vergleich zu der normalerweise in dem Pflanzengewebe vorliegenden Konzentration verringert wird.

10. Verfahren nach Anspruch 9, wobei die DNA-Sequenz in einem Konstrukt nach einem der Ansprüche 1 bis 8 enthalten ist.

11. Verfahren nach Anspruch 10, wobei das Enzym aus *Pseudomonas sp.* ACP erhalten werden kann.

12. Verfahren nach einem der Ansprüche 9 bis 11, wobei die DNA-Sequenz unter der Transkriptions- und Translationsinitiationskontrolle eines Kronblatt-spezifischen, Laubblatt-spezifischen, Wurzelspitzen-spezifischen oder Frucht-spezifischen Promotors steht.

13. Pflanzenzelle, umfassend ein DNA-Konstrukt nach einem der Ansprüche 1 bis 8.

14. Pflanzenzelle, umfassend eine bakterielle 1-Aminocyclopropan-1-carbonsäure (ACC)-desaminase.

15. Verfahren zum Herstellen einer 1-Aminocyclopropancarbonsäure(ACC)-desaminase, wobei die Desaminase in der Lage ist, 1-Aminocyclopropan-1-carbonsäure (ACC) zu metabolisieren und somit die Konzentration an Ethylen, die in einer Wirtszelle vorliegt, zu verringern, wobei das Verfahren umfaßt:
eine Wirtszelle mit einem DNA-Konstrukt nach einem der Ansprüche 1 bis 8 zu transformieren und die Zelle unter Bedingungen zu züchten, die die Produktion der Desaminase erlauben.

16. Verfahren nach Anspruch 15, wobei die Wirtszelle eine Pflanzenzelle ist und das Konstrukt in das Genom der Pflanzenzelle integriert wird.

17. Verfahren nach Anspruch 16, wobei die Pflanzenzelle sich *in vivo* befindet.

18. Pflanzenzelle, umfassend ein Enzym, wie in Anspruch 15 definiert.

19. Pflanzenzelle mit einer verringerten Ethylenkonzentration im Vergleich zu der normalerweise in der Zelle vorliegenden Konzentration, die durch das Verfahren nach einem der Ansprüche 9 bis 12 oder 16 erhalten werden kann.

20. Verfahren zum Verändern von Ethylenkonzentrationen in einer Pflanze, welches umfaßt:
eine Pflanzenzelle mit einer 1-Aminocyclopropancarbonsäure (ACC)-desaminase kodierenden DNA-Sequenz zu transformieren, eine Pflanze zu erhalten, die eine oder mehrere Zellen, umfassend die DNA-Sequenz, umfaßt, und die DNA-Sequenz in der Pflanze zu exprimieren, wobei eine Expression der DNA-Sequenz die für eine Ethylenumwandlung in der Pflanze zur Verfügung stehende ACC verringert.

21. Verfahren nach Anspruch 20, wobei die DNA-Sequenz innerhalb eines Konstrukts nach einem der Ansprüche 1 bis 8 enthalten ist.

## Patentansprüche (Patentansprüche für folgende(n) Vertragsstaat(en): ES)

1. Verfahren zum Verringern von Ethylenkonzentrationen in Pflanzengewebe, welches umfaßt: eine Pflanzenzelle mit einer 1-Aminocyclopropancarbonsäure (ACC)-desaminase kodierenden DNA-Sequenz zu transformieren, um dadurch Pflanzengewebe zu erhalten, das eine oder mehrere Pflanzenzellen umfaßt, die eine eine 1-Aminocyclopropan-1-carbonsäure(ACC)-desaminase kodierende DNA-Sequenz umfaßt bzw. umfassen, wobei die Desaminase in der Lage ist, 1-Aminocyclopropan-1-carbonsäure (ACC) zu metabolisieren und somit die Ethylenbiosynthese zu verringern, und das Gewebe unter Bedingungen zu züchten, wodurch das Gen exprimiert wird und die Ethylenkonzentration des Pflanzengewebes im Vergleich zu der normalerweise in dem Pflanzengewebe vorliegenden Konzentration verringert wird.

2. Verfahren nach Anspruch 1, wobei die DNA-Sequenz in einem DNA-Konstrukt enthalten ist, das umfaßt:
in der 5'-3'-Richtung der Transkription die folgenden Komponenten: einen in einer Pflanzenzelle funktionellen Transkriptions- und Translationsinitiationsbereich, eine eine 1-Aminocyclopropan-1-carbonsäure(ACC)-desaminase kodierende DNA-Sequenz, wobei die Desaminase in der Lage ist, 1-Aminocyclopropan-1-carbonsäure (ACC) zu metabolisieren und somit die Ethylenbiosynthese zu verringern, und einen Transkriptionsterminationsbereich, wobei die Komponenten in operabler oder funktioneller Weise verknüpft sind und wobei mindestens einer der Initiations- und Terminationsbereiche nicht der natürlicherweise mit der DNA-Sequenz assoziierte Bereich ist.

3. Verfahren nach Anspruch 2, wobei die DNA-Sequenz eine bakterielle 1-Aminocyclopropan-1-carbonsäure (ACC)-desaminase kodiert.

4. Verfahren nach Anspruch 3, wobei die die ACC-Desaminase kodierende DNA-Sequenz aus *Pseudomonas sp.* ACP erhalten werden kann.

5. Verfahren nach Anspruch 4, wobei die DNA-Sequenz *Pseudomonas sp.* ACP ACC-Desaminase, wie in Figur 2 gezeigt, kodiert.

6. Verfahren nach einem der Ansprüche 2 bis 5, wobei das Konstrukt ferner ein prokaryotisches Breitspektrumreplikationssystem umfaßt.

7. Verfahren nach einem der Ansprüche 2 bis 6, wobei das Konstrukt ferner einen Bereich von Homologie an mindestens dem rechten T-DNA-Rand mit einer ausreichenden Größe umfaßt, um eine Integration in ein Pflanzengenom zu ermöglichen, wobei der Bereich mit dem Konstrukt verknüpft ist.

8. Verfahren nach einem der Ansprüche 2 bis 7, wobei der Transkriptions- und Translationsinitiationsbereich einen doppelten 35S-CaMV-Promotor umfaßt.

9. Verfahren nach einem der Ansprüche 2 bis 7, wobei der Translationsinitiationsbereich einen Kronblatt-spezifischen, Laubblatt-spezifischen, Wurzelspitzen-spezifischen oder Frucht-spezifischen Promotor umfaßt.

10. Verfahren zum Herstellen einer 1-Aminocyclopropancarbonsäure (ACC)-desaminase, wobei die Desaminase in der Lage ist, 1-Aminocyclopropan-1-carbonsäure (ACC) zu metabolisieren und somit die Konzentration an Ethylen, die in einer Wirtszelle vorliegt, zu verringern, wobei das Verfahren umfaßt:
eine Wirtszelle mit einem DNA-Konstrukt, wie in einem der Ansprüche 2 bis 9 definiert, zu transformieren und die Zelle unter Bedingungen zu züchten, die die Produktion der Desaminase erlauben.

11. Verfahren nach Anspruch 10, wobei die Wirtszelle eine Pflanzenzelle ist und das Konstrukt in das Genom der Pflanzenzelle integriert wird.

12. Verfahren nach Anspruch 11, wobei die Pflanzenzelle sich *in vivo* befindet.

13. Verfahren zum Verändern von Ethylenkonzentrationen in einer Pflanze, welches umfaßt:
eine Pflanzenzelle mit einer 1-Aminocyclopropancarbonsäure (ACC)-desaminase kodierenden DNA-Sequenz zu transformieren, eine Pflanze zu erhalten, die eine oder mehrere Zellen, die die DNA-Sequenz umfaßt bzw. umfassen, umfaßt, und die DNA-Sequenz in der Pflanze zu exprimieren, wobei eine Expression der DNA-Se-quenz die für eine Ethylenumwandlung in der Pflanze zur Verfügung stehende ACC verringert.

14. Verfahren nach Anspruch 13, wobei die DNA-Sequenz innerhalb eines Konstrukts, wie in einem der Ansprüche 2 bis 9 definiert, enthalten ist.

15. DNA-Konstrukt, umfassend:
in der 5'-3'-Richtung der Transkription die folgenden Komponenten: einen Transkriptions- und Translationsinitiationsbereich, eine eine 1-Aminocyclopropan-1-carbonsäure(ACC)-desaminase kodierende DNA-Sequenz, wobei die Desaminase in der Lage ist, 1Aminocyclopropan-1-carbonsäure (ACC) zu metabolisieren und somit die Ethylenbiosynthese zu verringern, und einen Transkriptionsterminationsbereich, wobei die Komponenten in operabler oder funktioneller Weise verknüpft sind und wobei mindestens einer der Initiations- und Terminationsbereiche nicht der natürlicherweise mit der DNA-Sequenz assoziierte Bereich ist.

16. DNA-Konstrukt nach Anspruch 15, wobei die DNA-Sequenz eine bakterielle 1-Aminocyclopropan-1-carbonsäure(ACC)-desaminase kodiert.

17. DNA-Konstrukt nach Anspruch 16, wobei die die ACC-Desaminase kodierende DNA-Sequenz aus *Pseudomonas sp.* ACP erhalten werden kann.

18. DNA-Konstrukt nach Anspruch 17, wobei die DNA-Sequenz *Pseudomonas sp.* ACP ACC-Desaminase, wie in Figur 2 gezeigt, kodiert.

19. DNA-Konstrukt nach einem der Ansprüche 15 bis 18, das ferner ein prokaryotisches Breitspektrumreplikationssystem umfaßt.

20. DNA-Konstrukt nach einem der Ansprüche 15 bis 19, das ferner einen Bereich von Homologie an mindestens dem rechten T-DNA-Rand mit einer ausreichenden Größe umfaßt, um eine Integration in ein Pflanzengenom zu ermöglichen, wobei der Bereich mit dem Konstrukt verknüpft ist.

21. DNA-Konstrukt nach einem der Ansprüche 15 bis 20, wobei der Transkriptions- und Translationsinitiationsbereich einen doppelten 35S-CaMV-Promotor umfaßt.

22. DNA-Konstrukt nach einem der Ansprüche 15 bis 21, wobei der Translationsinitiationsbereich einen Kronblatt-spezifischen, Laubblatt-spezifischen, Wurzelspitzen-spezifischen oder Frucht-spezifischen Promotor umfaßt.

23. Pflanzenzelle, umfassend ein DNA-Konstrukt nach einem der Ansprüche 15 bis 22.

24. Pflanzenzelle, umfassend eine bakterielle 1-Aminocyclopropan-1-carbonsäure(ACC)-desaminase.

25. Pflanzenzelle, umfassend eine 1-Aminocyclopropancarbonsäure (ACC)-desaminase, wobei die Desaminase in der Lage ist, 1-Aminocyclopropan-1-carbonsäure (ACC) zu metabolisieren und somit die Konzentration an Ethylen, die in einer Wirtszelle vorliegt, zu verringern.

26. Pflanzenzelle mit einer verringerten Ethylenkonzentration im Vergleich zu der normalerweise in der Zelle vorliegenden Konzentration, die durch das Verfahren nach einem der Ansprüche 1 bis 9 oder 11 erhalten werden kann.

## Revendications (Revendications pour l'(les) Etat(s) contractant(s) suivant(s): AT, BE, CH, DE, DK, FR, GB, GR, IT, LI, LU, NL, SE)

1. Construction d'ADN qui comprend, dans la direction 5'-3' de transcription, les composants suivants :
- une région d'initiation de transcription et de traduction, qui peut fonctionner dans une cellule végétale ;
- une séquence d'ADN codant une 1-aminocyclopropane-1-carboxylate désaminase (ACC désaminase), qui est capable de métaboliser l'acide 1-aminocyclopropane-1-carboxylique (ACC) et de réduire ainsi la biosynthèse d'éthylène ;
- et une région de terminaison de transcription ;
et dans laquelle ces composants sont opérationnellement liés, et l'une au moins desdites régions d'initiation et de terminaison est différente de la région naturellement associée à ladite séquence d'ADN.

2. Construction d'ADN, conforme à la revendication 1, dans laquelle la séquence d'ADN code une 1-aminocyclopropane-1-carboxylate désaminase (ACC désaminase) bactérienne.

3. Construction d'ADN, conforme à la revendication 2, dans laquelle ladite séquence d'ADN codant ladite ACC désaminase peut être obtenue à partir de *Pseudomonas sp.* ACP.

4. Construction d'ADN, conforme à la revendication 3, dans laquelle ladite séquence d'ADN code l'ACC désaminase de *Pseudomonas sp.* ACP qui est représentée sur la figure 2.

5. Construction d'ADN, conforme à l'une des revendications précédentes, qui comporte en outre un système de réplication à large spectre, provenant d'un procaryote.

6. Construction d'ADN, conforme à l'une des revendications précédentes, qui comporte en outre une région homologue à au moins la région frontière droite de l'ADN-T, d'une taille suffisante pour assurer l'intégration dans un génome végétal, ladite région étant jointe à ladite construction.

7. Construction d'ADN, conforme à l'une des revendications précédentes, dans laquelle ladite région d'initiation de transcription et de traduction comporte un double promoteur CaMV 35S.

8. Construction d'ADN, conforme à l'une des revendications 1 à 6, dans laquelle la région d'initiation de traduction comporte un promoteur spécifique des pétales, des feuilles, des pointes de racines ou des fruits.

9. Procédé permettant de réduire les taux d'éthylène dans des tissus végétaux, qui comporte :
- le fait de modifier génétiquement une cellule végétale, au moyen d'une séquence d'ADN codant une 1-aminocyclopropane-1-carboxylate désaminase (ACC désaminase), de manière à obtenir un tissu végétal qui comprend une ou plusieurs cellules végétales contenant une séquence d'ADN qui code une 1-aminocyclopropane-1-carboxylate désaminase (ACC désaminase), laquelle désaminase est capable de métaboliser l'acide 1-aminocyclopropane-1-carboxylique (ACC) et de réduire ainsi la biosynthèse d'éthylène ;
- et le fait de faire proliférer ledit tissu dans des conditions telles que ledit gène s'exprime et que le taux d'éthylène dans ledit tissu végétal devient inférieur au taux constaté normalement dans ce tissu végétal.

10. Procédé conforme à la revendication 9, dans lequel ladite séquence d'ADN est contenue dans une construction conforme à l'une des revendications 1 à 8.

11. Procédé conforme à la revendication 10, dans lequel ladite enzyme peut être obtenue à partir de *Pseudomonas sp.* ACP.

12. Procédé conforme à l'une des revendications 9 à 11, dans lequel ladite séquence d'ADN se trouve, pour l'initiation de la transcription et de la traduction, sous le contrôle d'un promoteur spécifique des pétales, des feuilles, des pointes de racines ou des fruits.

13. Cellule végétale qui contient une construction d'ADN conforme à l'une des revendications 1 à 8.

14. Cellule végétale contenant une 1-aminocyclopropane-1-carboxylate désaminase (ACC désaminase) bactérienne.

15. Procédé permettant de produire une 1-aminocyclopropane-1-carboxylate désaminase (ACC désaminase) capable de métaboliser l'acide 1-aminocyclopropane-1-carboxylique (ACC), et de réduire ainsi le taux d'éthylène présent dans une cellule hôte, lequel procédé comporte le fait de modifier génétiquement une cellule hôte au moyen d'une construction d'ADN conforme à l'une des revendications 1 à 8, et le fait de faire proliférer cette cellule dans des conditions qui permettent la production de ladite désaminase.

16. Procédé conforme à la revendication 15, dans lequel ladite cellule hôte est une cellule végétale, et ladite construction est intégrée dans le génome de cette cellule végétale.

17. Procédé conforme à la revendication 16, dans lequel ladite cellule végétale se trouve *in vivo.*

18. Cellule végétale qui contient une enzyme du type défini dans la revendication 15.

19. Cellule végétale contenant de l'éthylène à un taux inférieur à celui normalement constaté dans cette cellule, qu'on peut obtenir selon un procédé conforme à l'une des revendications 9 à 12 et 16.

20. Procédé permettant de modifier les taux d'éthylène chez un végétal, qui comporte :
- le fait de modifier génétiquement une cellule végétale, au moyen d'une séquence d'ADN codant une 1-aminocyclopropane-1-carboxylate désaminase (ACC désaminase) ;
- le fait d'obtenir un végétal dont une ou plusieurs des cellules contiennent ladite séquence d'ADN ;
- et le fait de faire s'exprimer ladite séquence d'ADN chez ledit végétal ;
l'expression de cette séquence d'ADN ayant pour effet de réduire, chez ledit végétal, la quantité d'ACC disponible pour la conversion en éthylène.

21. Procédé conforme à la revendication 20, dans lequel ladite séquence d'ADN est contenue dans une construction conforme à l'une des revendications 1 à 8.

## Revendications (Revendications pour l'(les) Etat(s) contractant(s) suivant(s): ES)

1. Procédé permettant de réduire les taux d'éthylène dans des tissus végétaux, qui comporte :
- le fait de modifier génétiquement une cellule végétale, au moyen d'une séquence d'ADN codant une 1-aminocyclopropane-1-carboxylate désaminase (ACC désaminase), de manière à obtenir un tissu végétal qui comprend une ou plusieurs cellules végétales contenant une séquence d'ADN qui code une 1-aminocyclopropane-1-carboxylate désaminase (ACC désaminase), laquelle désaminase est capable de métaboliser l'acide 1-aminocyclopropane-1-carboxylique (ACC) et de réduire ainsi la biosynthèse d'éthylène ;
- et le fait de faire proliférer ledit tissu dans des conditions telles que ledit gène s'exprime et que le taux d'éthylène dans ledit tissu végétal devient inférieur au taux constaté normalement dans ce tissu végétal.

2. Procedé conforme à la revendication 1, dans lequel ladite séquence d'ADN se trouve dans une construction d'ADN qui comprend, dans la direction 5'-3' de transcription, les composants suivants :
- une région d'initiation de transcription et de traduction, qui peut fonctionner dans une cellule végétale ;
- une séquence d'ADN codant une 1-aminocyclopropane-1-carboxylate désaminase (ACC désaminase), qui est capable de métaboliser l'acide 1-aminocyclopropane-1-carboxylique (ACC) et de réduire ainsi la biosynthèse d'éthylène ;
- et une région de terminaison de transcription ;
et dans laquelle ces composants sont opérationnellement liés, et l'une au moins desdites régions d'initiation et de terminaison est différente de la région naturellement associée à ladite séquence d'ADN.

3. Procédé conforme à la revendication 2, dans lequel ladite séquence d'ADN code une 1-aminocyclopropane-1-carboxylate désaminase (ACC désaminase) bactérienne.

4. Procédé conforme à la revendication 3, dans lequel ladite séquence d'ADN codant ladite ACC désaminase peut être obtenue à partir de *Pseudomonas sp.* ACP.

5. Procédé conforme à la revendication 4, dans lequel ladite séquence d'ADN code l'ACC désaminase de *Pseudomonas sp.* ACP qui est représentée sur la figure 2.

6. Procédé conforme à l'une des revendications 2 à 5, dans lequel ladite construction comporte en outre un système de réplication à large spectre, provenant d'un procaryote.

7. Procédé conforme à l'une des revendications 2 à 6, dans lequel ladite construction comporte en outre une région homologue à au moins la région frontière droite de l'ADN-T, d'une taille suffisante pour assurer l'intégration dans un génome végétal, ladite région étant jointe à ladite construction.

8. Procédé conforme à l'une des revendications 2 à 7, dans lequel ladite région d'initiation de transcription et de traduction comporte un double promoteur CaMV 35S.

9. Procédé conforme à l'une des revendications 2 à 7, dans lequel la région d'initiation de traduction comporte un promoteur spécifique des pétales, des feuilles, des pointes de racines ou des fruits.

10. Procédé permettant de produire une 1-aminocyclopropane-1-carboxylate désaminase (ACC désaminase) capable de métaboliser l'acide 1-aminocyclopropane-1-carboxylique (ACC), et de réduire ainsi le taux d'éthylène présent dans une cellule hôte, lequel procédé comporte le fait de modifier génétiquement une cellule hôte au moyen d'une construction d'ADN du type défini dans l'une des revendications 2 à 9, et le fait de faire proliférer cette cellule dans des conditions qui permettent la production de ladite désaminase.

11. Procédé conforme à la revendication 10, dans lequel ladite cellule hôte est une cellule végétale, et ladite construction est intégrée dans le génome de cette cellule végétale.

12. Procédé conforme à la revendication 11, dans lequel ladite cellule végétale se trouve *in vivo.*

13. Procédé permettant de modifier les taux d'éthylène chez un végétal, qui comporte :
- le fait de modifier génétiquement une cellule végétale, au moyen d'une séquence d'ADN codant une 1-aminocyclopropane-1-carboxylate désaminase (ACC désaminase) ;
- le fait d'obtenir un végétal dont une ou plusieurs des cellules contiennent ladite séquence d'ADN ;
- et le fait de faire s'exprimer ladite séquence d'ADN chez ledit végétal ;
l'expression de cette séquence d'ADN ayant pour effet de réduire, chez ledit végétal, la quantité d'ACC disponible pour la conversion en éthylène.

14. Procédé conforme à la revendication 13, dans lequel ladite séquence d'ADN est contenue dans une construction du type défini dans l'une des revendications 2 à 9.

15. Construction d'ADN qui comprend, dans la direction 5'-3' de transcription, les composants suivants :
- une région d'initiation de transcription et de traduction ;
- une séquence d'ADN codant une 1-aminocyclopropane-1-carboxylate désaminase (ACC désaminase), qui est capable de métaboliser l'acide 1-aminocyclopropane-1-carboxylique (ACC) et de réduire ainsi la biosynthèse d'éthylène ;
- et une région de terminaison de transcription ;
et dans laquelle ces composants sont opérationnellement liés, et l'une au moins desdites régions d'initiation et de terminaison est différente de la région naturellement associée à ladite séquence d'ADN.

16. Construction d'ADN, conforme à la revendication 15, dans laquelle la séquence d'ADN code une 1-aminocyclopropane-1-carboxylate désaminase (ACC désaminase) bactérienne.

17. Construction d'ADN, conforme à la revendication 16, dans laquelle ladite séquence d'ADN codant ladite ACC désaminase peut être obtenue à partir de *Pseudomonas sp.* ACP.

18. Construction d'ADN, conforme à la revendication 17, dans laquelle ladite séquence d'ADN code l'ACC désaminase de *Pseudomonas sp.* ACP qui est représentée sur la figure 2.

19. Construction d'ADN, conforme à l'une des revendications 15 à 18, qui comporte en outre un système de réplication à large spectre, provenant d'un procaryote.

20. Construction d'ADN, conforme à l'une des revendications 15 à 19, qui comporte en outre une région homologue à au moins la région frontière droite de l'ADN-T, d'une taille suffisante pour assurer l'intégration dans un génome végétal, ladite région étant jointe à ladite construction.

21. Construction d'ADN, conforme à l'une des revendications 15 à 20, dans laquelle ladite région d'initiation de transcription et de traduction comporte un double promoteur CaMV 35S.

22. Construction d'ADN, conforme à l'une des revendications 15 à 21, dans laquelle la région d'initiation de traduction comporte un promoteur spécifique des pétales, des feuilles, des pointes de racines ou des fruits.

23. Cellule végétale qui contient une construction d'ADN conforme à l'une des revendications 15 à 22.

24. Cellule végétale contenant une 1-aminocyclopropane-1-carboxylate désaminase (ACC désaminase) bactérienne.

25. Cellule végétale contenant une 1-aminocyclopropane-1carboxylate désaminase (ACC désaminase) qui est capable de métaboliser l'acide 1-aminocyclopropane-1-carboxylique (ACC) et de réduire ainsi le taux d'éthylène présent dans une cellule hôte.

26. Cellule végétale contenant de l'éthylène à un taux inférieur à celui normalement constaté dans cette cellule, qu'on peut obtenir selon un procédé conforme à l'une des revendications 1 à 9 et 11.


### SEQUENCE LISTING

### (1) GENERAL INFORMATION:

(i) APPLICANT:
   Shang Fa Yang
   William R. Hiatt
(ii) TITLE OF INVENTION:
   Methods and Compositions For Modulating Ethylene Levels In Plant Tissues
(iii) NUMBER OF SEQUENCES: 9
(iv) CORRESPONDENCE ADDRESS:
   (A) ADDRESSEE: Calgene, Inc.
   (B) STREET: 1920 Fifth Street
   (C) CITY: Davis
   (D) STATE: CA
   (E) COUNTRY: USA
   (F) ZIP: 95616
(v) COMPUTER READABLE FORM:
   (A) MEDIUM TYPE: Diskette, 3.50 inch, 1.0MB
   (B) COMPUTER: Apple Macintosh
   (C) OPERATING SYSTEM: Macintosh 6.0
   (D) SOFTWARE: Microsoft Word 4.0
(vi) CURRENT APPLICATION DATA:
   (A) APPLICATION NUMBER: unassigned
   (B) FILING DATE:
   (C) CLASSIFICATION:
(vii) PRIOR APPLICATION DATA:
   (A) APPLICATION NUMBER: USSN 07/514,029
   (B) FILING DATE: 26-APR-90
(viii) ATTORNEY/AGENT INFORMATION:
   (A) NAME: Elizabeth Lassen
   (B) REGISTRATION NUMBER: 31,845
   (A) NAME: Donna E. Scherer
   (B) REGISTRATION NUMBER: P-34,719
   (C) REFERENCE/DOCKET NUMBER: CGNE 67-1 WO
(ix) TELECOMMUNICATION INFORMATION:
   (A) TELEPHONE: 916-753-6313
   (B) TELEFAX: 916-753-1510
(2) INFORMATION FOR SEQ ID NO: 1:
   (i) SEQUENCE CHARACTERISTICS:
      (A) LENGTH: 8 amino acids
      (B) TYPE: amino acid
         (D) TOPOLOGY: linear
   (ii) MOLECULE TYPE: peptide
   (xi) SEQUENCE DESCRIPTION: SEQ ID NO: 1:
   Met Asn Leu Gln Arg Phe Pro Arg  8
    1               5

### (2) INFORMATION FOR SEQ ID NO: 2:

(i) SEQUENCE CHARACTERISTICS:
   (A) LENGTH: 8 amino acids
   (B) TYPE: amino acid
      (D) TOPOLOGY: linear
(ii) MOLECULE TYPE: peptide
(xi) SEQUENCE DESCRIPTION: SEQ ID NO: 2:
   Gln Glu Asn Trp Val Asn Tyr Ser  8
    1               5

### (2) INFORMATION FOR SEQ ID NO: 3:

(i) SEQUENCE CHARACTERISTICS:
   (A) LENGTH: 8 amino acids
   (B) TYPE: amino acid
      (D) TOPOLOGY: linear
(ii) MOLECULE TYPE: peptide
(xi) SEQUENCE DESCRIPTION: SEQ ID NO: 3:
   Arg Val Gly Asn Ile Gln Met Ser  8
    1               5

### (2) INFORMATION FOR SEQ ID NO: 4:

(i) SEQUENCE CHARACTERISTICS:
   (A) LENGTH: 7 amino acids
   (B) TYPE: amino acid
      (D) TOPOLOGY: linear
(ii) MOLECULE TYPE: peptide
(xi) SEQUENCE DESCRIPTION: SEQ ID NO: 4:
   Val Pro Asp Thr Phe Asp Thr  7
    1               5

### (2) INFORMATION FOR SEQ ID NO: 5:

(i) SEQUENCE CHARACTERISTICS:
   (A) LENGTH: 32 base pairs
   (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 5:
   CGTATCTAGA TGAAYYTNCA RMGNTTYCCN MG 32

### (2) INFORMATION FOR SEQ ID NO: 6:

(i) SEQUENCE CHARACTERISTICS:
   (A) LENGTH: 34 base pairs
   (B) TYPE: nucleic acid
      (C) STRANDEDNESS: single
      (D) TOPOLOGY: linear
(ii) MOLECULE TYPE: other nucleic acid
(xi) SEQUENCE DESCRIPTION: SEQ ID NO: 6:
   GCATGTCGAC ARGARAAYTG GGTNAAYTAY WSNG 34

### (2) INFORMATION FOR SEQ ID NO: 7:

(i) SEQUENCE CHARACTERISTICS:
   (A) LENGTH: 32 base pairs
   (B) TYPE: nucleic acid
      (C) STRANDEDNESS: single
      (D) TOPOLOGY: linear
(ii) MOLECULE TYPE: other nucleic acid
(xi) SEQUENCE DESCRIPTION: SEQ ID NO: 7:
   CANCCNTTRT ADGTYTACWS NKCTTAAGCA CT 32

### (2) INFORMATION FOR SEQ ID NO: 8:

(i) SEQUENCE CHARACTERISTICS:
   (A) LENGTH: 30 base pairs
   (B) TYPE: nucleic acid
      (C) STRANDEDNESS: single
      (D) TOPOLOGY: linear
(ii) MOLECULE TYPE: other nucleic acid
(xi) SEQUENCE DESCRIPTION: SEQ ID NO: 8:
   CANGGNCTRT GNAARCTRTG TTCGAATAGC   30

### (2) INFORMATION FOR SEQ ID NO: 9:

(i) SEQUENCE CHARACTERISTICS:
   (A) LENGTH: 1778 base pairs
   (B) TYPE: nucleic acid
      (C) STRANDEDNESS: single
      (D) TOPOLOGY: linear
(ii) MOLECULE TYPE: genomic DNA
(xi) SEQUENCE DESCRIPTION: SEQ ID NO: 9:
   GAATTCTCCG ATCTCGCATG TCGCGAGGAG GGTTATGGGT TCGCCCAAGG CCCGCAAATG    60
   GATAAAAACG GCCCCGGGCG GCCGCCGCTC TGTCAGGTGC GGCATGTAAC TTTTTCAGCG   120
   AACNGCACGC GCTGTCCGGC AAAACACACG ACTTGCACGC GGTCGATGGT CCGTCAGACG   180
   GACATTTGTC TAACCAGACC GGTGCGAAAG ATTTGCCGAT GGATGCATCT TTGCAAAATG   240
   CGGGCCCGAG CAGTGGAGCG TAGGTTCGGA TTGCGCCGGC GCGGCCGGCT TGACCGTGGC   300
   CGCGAGCGAG ACATGTGCAA CTGACGCGCC GGCTTTCAAC CGCCTCGAGA TCGCCCGATC   360
   GAGGCGCTCG ATCGATTGCA GCGGACCTGC CAGCACTGCA ACGGCAAGGG TAAGGGCAAC   420
   CGCAGTCCGA CTTGGGTCGT CACGAATTTT TGTTGCATCC ATAACCATCT TTACCAATCC   480
   TTTGTTAATT CAAATTGCCA TACCTATTTT CAAAATTATG TCTAGTTAAA TTTTTTCGCA   540
   AGCACATTTC CAATGCATTT GCCTACCATT TCTATACCGC GACGGATGCT GTTCGCGTCT   600
   CTTTTACCGA TCAAGGAGCT GCC ATG AAC CTG CAA CGA TTC CCT CGT TAC CCG   653
                             Met Asn Leu Gln Arg Phe Pro Arg Tyr Pro
   CTG ACT TTC GGG CCG ACG CCA ATC CAA CCG CTA GCG CGT CTG AGC AAG     701
   Leu Thr Phe Gly Pro Thr Pro Ile Gln Pro Leu Ala Arg Leu Ser Lys
   CAC CTC GGC GGC AAA GTG CAT CTG TAT GCG AAA CGC GAA GAC TGC AAC     749
   His Leu Gly Gly Lys Val His Leu Tyr Ala Lys Arg Glu Asp Cys Asn
   AGC GGC CTG GCG TTC GGT GGC AAC AAG ACA CGC AAG CTC GAA TAT CTG     797
   Ser Gly Leu Ala Phe Gly Gly Asn Lys Thr Arg Lys Leu Glu Tyr Leu
   ATC CCT GAA GCG CTT GCT CAG GGT TGC GAC ACG CTC GTG TCG ATC GGC     845
   Ile Pro Glu Ala Leu Ala Gln Gly Cys Asp Thr Leu Val Ser Ile Gly
   GGC ATT CAG TCG AAC CAG ACG CGC CAG GTG GCG GCC GTG GCG GCT CAT     893
   Gly Ile Gln Ser Asn Gln Thr Arg Gln Val Ala Ala Val Ala Ala His
   CTG GGC ATG AAG TGC GTG CTG GTG CAG GAG AAC TGG GTC AAC TAT TCG     941
   Leu Gly Met Lys Cys Val Leu Val Gln Glu Asn Trp Val Asn Tyr Ser
   GAC GCA GTC TAC GAC CGC GTC GGC AAC ATC CAG ATG TCG CGC ATT CTC     989
   Asp Ala Val Tyr Asp Arg Val Gly Asn Ile Gln Met Ser Arg Ile Leu
   GGC GCC GAT GTT CGC CTC GTG CCC GAC GGC TTC GAC ATC GGT TTT CGC    1037
   Gly Ala Asp Val Arg Leu Val Pro Asp Gly Phe Asp Ile Gly Phe Arg
   AGG AGC TGG GAG GAT GCG CTG GAA AGC GTG CGG GCG GCC GGC GGC AAG    1085
   Arg Ser Trp Glu Asp Ala Leu Glu Ser Val Arg Ala Ala Gly Gly Lys
   CCG TAT GCG ATT CCG GCA GGC TGC TCG GAT CAC CCG CTC GGC GGC CTG    1133
   Pro Tyr Ala Ile Pro Ala Gly Cys Ser Asp His Pro Leu Gly Gly Leu
   GGT TTC GTC GGC TTC GCG GAG GAG GTG CGG GCG CAG GAA GCC GAA TTG    1181
   Gly Phe Val Gly Phe Ala Glu Glu Val Arg Ala Gln Glu Ala Glu Leu
   GGC TTC AAA TTC GAC TAT GTC GTC GTG TGT TCC GTG ACC GGC AGC ACG    1229
   Gly Phe Lys Phe Asp Tyr Val Val Val Cys Ser Val Thr Gly Ser Thr
   CAG GCC GGC ATG GTG GTG GGA TTC GCC GCT GAC GGC CGN GCC GAT CGC    1277
   Gln Ala Gly Met Val Val Gly Phe Ala Ala Asp Gly Arg Ala Asp Arg
   GTG ATC GGC GTC GAC GCT TCG GCC AAA CCC GCG CAG ACG CGC GAG CAG    1325
   Val Ile Gly Val Asp Ala Ser Ala Lys Pro Ala Gln Thr Arg Glu Gln
   ATC ACC CNC ATC GCG AGA CAG ACC GCG GAG AAA GTC GGC CTG GAG CGC    1373
   Ile Thr Xaa Ile Ala Arg Gln Thr Ala Glu Lys Val Gly Leu Glu Arg
   GAT ATC ATG CGG GCC GAC GTG GTG CTC GAC GAG CGC TTC GCG GGT CCG    1421
   Asp Ile Met Arg Ala Asp Val Val Leu Asp Glu Arg Phe Ala Gly Pro
   GAA TAC GGA TTG CCG AAC GAA GGC ACG CTG GAA GCG ATC CGC TTG TGC    1469
   Glu Tyr Gly Leu Pro Asn Glu Gly Thr Leu Glu Ala Ile Arg Leu Cys
   GCG CGC ACG GAG GGC ATG CTG ACC GAT CCC GTC TAC GAA GGC AAA TCG    1517
   Ala Arg Thr Glu Gly Met Leu Thr Asp Pro Val Tyr Glu Gly Lys Ser
   ATG CAC GGC ATG ATC GAA ATG GTG CGC AAC GGC GAA TTT CCG GAA GGC    1565
   Met His Gly Met Ile Glu Met Val Arg Asn Gly Glu Phe Pro Glu Gly
   TCG CGC GTG CTG TAT GCG CAC CTC GGC GGG GTG CCG GCG TTG AAC GGC    1613
   Ser Arg Val Leu Tyr Ala His Leu Gly Gly Val Pro Ala Leu Asn Gly
   TAC AGC TTT ATC TTC CGA GAC GGC TGAACGCTCC GACCGGCGGC CAGCACCGGC  1667
   Tyr Ser Phe Ile Phe Arg Asp Gly
   AAGCAGCGAC CGGCGCCTGT CCAGCCGGTC NCTCNTTTAC TCGTGCGCTT TACTCGTGCG 1727
   CTTTACTCGT GCGCTTTACT CGTGCGCTTT ACTCGTGCGG TTGCACCGGA T          1778